(19) **Europäisches Patentamt**
**European Patent Office**
**Office européen des brevets**

(11) **EP 3 174 596 B1**

(12) **EUROPEAN PATENT SPECIFICATION**

(45) Date of publication and mention of the grant of the patent:
**08.03.2023   Bulletin 2023/10**

(21) Application number: **15753248.2**

(22) Date of filing: **30.07.2015**

(51) International Patent Classification (IPC):
**G16H 20/40** (2018.01)      **G16H 50/70** (2018.01)
**G16H 30/20** (2018.01)      **A61N 1/36** (2006.01)
**A61N 1/05** (2006.01)      **A61N 1/372** (2006.01)

(52) Cooperative Patent Classification (CPC):
**A61N 1/36025; A61N 1/37241; G16H 20/40;**
**G16H 30/20; G16H 50/70;** A61N 1/0534;
A61N 1/36132; G16H 10/65; G16H 50/30

(86) International application number:
**PCT/US2015/042873**

(87) International publication number:
**WO 2016/019129 (04.02.2016 Gazette 2016/05)**

(54) **COMPUTER IMPLEMENTED METHOD FOR STIMULATION-RELATED VOLUME ANALYSIS, CREATION, AND SHARING WITH INTEGRATED SURGICAL PLANNING AND STIMULATION PROGRAMMING**

COMPUTERIMPLEMENTIERTES VERFAHREN ZUR STIMULATIONSASSOZIIERTEN VOLUMENANALYSE, ERZEUGUNG UND GEMEINSAMEN NUTZUNG MIT INTEGRIERTER CHIRURGISCHER PLANUNG UND STIMULATIONSPROGRAMMIERUNG

MÉTHODE IMPLÉMENTÉE PAR ORDINATEUR POUR L'ANALYSE, LA CRÉATION ET LE PARTAGE DE VOLUMES LIÉS À UNE STIMULATION AVEC PROGRAMMATION CHIRURGICALE ET PROGRAMMATION DE STIMULATION INTÉGRÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.07.2014   US 201462031075 P**
**30.07.2014   US 201462030655 P**

(43) Date of publication of application:
**07.06.2017   Bulletin 2017/23**

(73) Proprietor: **Boston Scientific Neuromodulation Corporation**
**Valencia, CA 91355 (US)**

(72) Inventors:
• **CARCIERI, Stephen**
  **Los Angeles, CA 90026 (US)**
• **STEINKE, G. Karl**
  **Valencia, CA 91354 (US)**
• **YOO, Peter J.**
  **Burbank, CA 91501 (US)**

• **MUSTAKOS, Richard**
  **Thousand Oaks, CA 91361 (US)**
• **BOKIL, Hemant**
  **Cambridge, MA 02139 (US)**
• **CARLTON, Keith R.**
  **Boston, MA 02114 (US)**
• **MOFFITT, Michael A.**
  **Valencia, CA 91354 (US)**

(74) Representative: **Vossius & Partner**
**Patentanwälte Rechtsanwälte mbB**
**Siebertstrasse 3**
**81675 München (DE)**

(56) References cited:
**KR-A- 20110 066 460      US-A1- 2006 017 749**
**US-A1- 2013 116 748      US-A1- 2013 116 929**

• CHATURVEDI A ET AL: "Patient-specific models of deep brain stimulation: Influence of field model complexity on neural activation predictions", BRAIN STIMULATION, ELSEVIER, AMSTERDAM, NL, vol. 3, no. 2, 1 April 2010 (2010-04-01), pages 65-77, XP026996444, ISSN: 1935-861X [retrieved on 2010-02-04]

## Description

### FIELD OF THE INVENTION

The present invention pertains to sharing of target volumes of activation.

### BACKGROUND

Electrical stimulation of an anatomical region, e.g., deep brain stimulation (DBS), such as of the thalamus or basal ganglia, is a clinical technique for the treatment of disorders such as essential tremor, Parkinson's disease (PD), and other physiological disorders. DBS may also be useful for traumatic brain injury and stroke. Pilot studies have also begun to examine the utility of DBS for treating dystonia, epilepsy, and obsessive-compulsive disorder.

A stimulation procedure, such as DBS, typically involves first obtaining preoperative images, e.g., of the patient's brain, such as by using a computed tomography (CT) scanner device, a magnetic resonance imaging (MRI) device, or any other imaging modality. This sometimes involves first affixing to the patient's skull spherical or other fiducial markers that are visible on the images produced by the imaging modality. The fiducial markers help register the preoperative images to the actual physical position of the patient in the operating room during the later surgical procedure.

After the preoperative images are acquired by the imaging modality, they are then loaded onto an image-guided surgical (IGS) workstation, and, using the preoperative images displayed on the IGS workstation, a neurosurgeon can select a target region, e.g., within the brain, an entry point, e.g., on the patient's skull, and a desired trajectory between the entry point and the target region. The entry point and trajectory are typically carefully selected to avoid intersecting or otherwise damaging certain nearby critical structures or vasculature, e.g., of the brain.

In the operating room, the physician marks the entry point on the patient's skull, drills a burr hole at that location, and affixes a trajectory guide device about the burr hole. The trajectory guide device includes a bore that can be aimed to obtain the desired trajectory to the target region. After aiming, the trajectory guide is locked to preserve the aimed trajectory toward the target region. After the aimed trajectory has been locked in using the trajectory guide, a microdrive introducer is used to insert the surgical instrument along the trajectory toward the target region, e.g., of the brain. The surgical instrument may include, among other things, a recording electrode leadwire, for recording intrinsic electrical signals, e.g., of the brain; a stimulation electrode leadwire, for providing electrical energy to the target region, e.g., of the brain; or associated auxiliary guidewires or guide catheters for steering a primary instrument toward the target region, e.g., of the brain.

The stimulation electrode leadwire, which typically includes multiple closely-spaced electrically independent stimulation electrode contacts, is then introduced to deliver the therapeutic stimulation to the target region, e.g., of the brain. The stimulation electrode leadwire is then immobilized, such as by using an instrument immobilization device located at the burr hole entry, e.g., in the patient's skull, in order for the DBS therapy to be subsequently performed.

The subthalamic nucleus (STN) represents the most common target for DBS technology. Clinically effective STN DBS for PD has typically used electrode contacts in the anterior-dorsal STN. However, STN DBS exhibits a low threshold for certain undesirable side effects, such as tetanic muscle contraction, speech disturbance and ocular deviation. Highly anisotropic fiber tracks are located about the STN. Such nerve tracks exhibit high electrical conductivity in a particular direction. Activation of these tracks has been implicated in many of the DBS side effects. However, there exists a limited understanding of the neural response to DBS. The three-dimensional (3-D) tissue medium near the DBS electrode typically includes both inhomogeneous and anisotropic characteristics. Such complexity makes it difficult to predict the particular volume of tissue influenced by DBS.

After the immobilization of the stimulation electrode leadwire, the actual stimulation therapy is often not initiated until after a time period of about two-weeks to one month has elapsed. This is due primarily to the acute reaction of the brain tissue to the introduced electrode leadwire (e.g., the formation of adjacent scar tissue), and stabilization of the patient's disease symptoms. At that time, a particular one or more of the stimulation electrode contacts is selected for delivering the therapeutic stimulation, and other stimulation parameters are adjusted to achieve an acceptable level of therapeutic benefit.

A system and method may estimate stimulation volumes, and display models of a patient anatomy and/or a stimulation leadwire, via which to graphically identify the estimated stimulation volumes and how they interact with various regions of the patient anatomy.

The systems and methods may be used to explore target regions of stimulation and stimulation therapies to determine which therapy regimen is best suited for a particular patient or group of patients.

### SUMMARY

Such exploration may result in much data over time for a particular patient and/or for a patient population. The present invention discloses a computer implemented method to improve the quality of such data, to manage such data, and to maximize use of, and facilitate efficient use of, such information. Any systems or system components disclosed hereinafter do not form part of the scope of the invention and are mentioned for illustrative purposes only.

The data may pertain to, for example, stimulation of a patient for deep brain stimulation (DBS) therapy and/or spinal cord stimulation (SCS) therapy. It may include graphical information, such as estimated volumes of activation (VOA), also referred to herein as a stimulation field model (SFM). It may include information used for rendering the SFMs, such as image registration and/or leadwire location data. It may further include information regarding the patient's condition, such as disease and medications taken, and/or reactions to an applied therapy. It may further include information concerning stimulation programs applied to the patient for the patient therapy. It may include target volumes selected for a patient, and/or volumes of estimated activation (VOA) for various stimulation parameters input for the patient. It may include information concerning how close the patient's anatomical images match to a standard atlas. It may further include analytics information as described below.

Various systems, system components, and/or program modules may be used for performance of various tasks associated with, or that provide an output usable for, providing therapeutic stimulation, generation of data regarding a therapy, and access to and transfer of therapy data. Examples of the present invention provide for communication and/or between the various systems, system components, and/or program modules.

The various methods described herein may be practiced, each alone, or in various combinations.

The present invention, in particular, relates to a computer-implemented method according to claim 1. The dependent claims relate to preferred embodiments of said method. Also disclosed below are various exemplary systems, devices, methods which are not covered by the wording of claim 1.

An example is directed to a processor, which may be implemented using any conventional processing circuit and device or combination thereof, e.g., a Central Processing Unit (CPU) of a Personal Computer (PC) or other workstation processor, to execute code provided, e.g., on a hardware computer-readable medium including any conventional memory device, to perform any of the methods described herein, alone or in combination. In certain examples, the processor may be embodied in a remote control device. The memory device may include any conventional permanent and/or temporary memory circuits or combination thereof, a non-exhaustive list of which includes Random Access Memory (RAM), Read Only Memory (ROM), Compact Disks (CD), Digital Versatile Disk (DVD), and magnetic tape.

An example is directed to a hardware computer-readable medium, e.g., as described above, having stored thereon instructions executable by a processor to perform the methods described herein.

An example is directed to a method, e.g., of a hardware component or machine, of transmitting instructions executable by a processor to perform the methods described herein.

The present invention relates to a computer-implemented method, comprising: obtaining user input of a clinical effect, wherein the clinical effect is either a therapeutic effect or a side effect; obtaining a plurality of estimated volumes of activation (VOA) associated with stimulations of a patient population, wherein a first group of the VOAs is associated with the clinical effect and a second group of the VOAs is not associated with the clinical effect; based on the VOAs, determining a target stimulation region, when the clinical effect is the therapeutic effect, or a side effect region, when the clinical effect is the side effect, by determining, on a voxel-by-voxel basis, that a voxel is in the target stimulation region when the voxel is included in statistically significantly more of the VOAs of the first group of VOAs than in the VOAs of the second group of VOAs; and outputting the determined target stimulation or side effect region..

In at least some embodiments, the method further includes transforming all of the VOAs into a common spatial reference space. In at least some embodiments, determining a target stimulation region or a side effect region includes selecting points for the target stimulation region or side effect region that belong to a threshold number or percentage of the VOAs. In at least some embodiments, the method further includes obtaining a rating for each of the VOAs with respect to the at least one clinical effect, wherein determining a target stimulation region or a side effect region includes selecting points for the target stimulation region or side effect region that have a threshold score based on the VOAs containing the point weighted by the rating of the VOA. In at least some embodiments, outputting the determined region includes outputting the determined region indicating probabilities that points within the region will contribute to the at least one clinical effect.

In at least some embodiments, the method further includes filtering the VOAs by a time of day when stimulation of the VOA was performed. In at least some embodiments, the method further includes filtering the VOAs by a medication used by a patient associated with the VOA. In at least some embodiments, the at least one clinical effect includes at least one therapeutic effect and at least one side effect. In at least some embodiments, determining a target stimulation region or a side effect region includes determining a target stimulation region based on the at least one therapeutic effect and determining a side effect region based on the at least one side effect.

Another example is a computer-implemented method including grouping, into a plurality of groups, volumes of activation (VOA) associated with stimulations of a patient population according to clinical effects; for at least one of the groups, identifying a biological circuit element over which a threshold amount of the VOAs overlie; and providing an output that identifies the biological circuit element as a target or side effect stimulation element.

In at least some examples, the biological circuit element includes a neurological fiber. In at least some examples, grouping VOAs includes forming a first group of VOAs associated with a side effect, wherein providing an output includes outputting the biological circuit element over which the threshold amount of the VOAs of the first group of VOAs overlie as a circuit element to avoid. In at least some examples, grouping VOAs includes forming a first group of VOAs associated with a therapeutic effect, wherein providing an output includes outputting the biological circuit element over which the threshold amount of the VOAs of the first group of VOAs overlie as a circuit element to target.

Yet another example is a computer-implemented method including for each of a plurality of population groups, provide characteristics of the population group and an atlas based on anatomical images of the population group, wherein the population groups have been identified by analyzing clinical effects data stored in association with volumes of activation (VOA) of a patient population for commonalities or disparities in clinical effect; obtaining characteristics of a new patient; identifying which of the population groups are associated with characteristics that are most similar to those of the patient; and registering the atlas of the identified population group to the new patient to generate a patient-specific atlas.

In at least some examples, providing characteristics and an atlas for each of the population groups includes analyzing clinical effects data stored in association with volumes of activation (VOA) of a patient population for commonalities or disparities in clinical effect; based on the analysis, grouping the patient population into the plurality of population groups; for each of the population groups, generating an atlas based on anatomical images of the population group; and for each of the population groups, identifying commonalities in characteristics of the patient population of the population group.

In at least some examples, the population groups are identified, at least in part, based on commonalties of a therapeutic effect. In at least some examples, the population groups are identified, at least in part, based on commonalties of a side effect. In at least some examples, the population groups are identified, at least in part, based on commonalties of a side effect volume. In at least some examples, identifying which of the population groups are associated with characteristics that are most similar to those of the patient includes comparing at least one image of the patient with images of the population groups and selecting a patient population group based on image similarity. In at least some examples, the method further includes outputting a target region for stimulation based, at least in part, on the VOAs of the population group identified as most similar to the patient.

A further example is a computer-implemented method including obtaining atlas registration information regarding a patient from a surgical planning module in which a surgical trajectory for implantation of a leadwire is generated; obtaining from the surgical planning module a target volume to be activated; determining, by a processor, stimulation parameter settings that are estimated to provide stimulation in at least a portion of the target volume; analyzing, by the processor, effects of stimulations performed on a plurality of patients of a patient population; based on the analysis, determining, by the processor, a new target volume; and providing the new target volume for access by the surgical planning module.

In at least some examples, obtaining atlas registration information includes identifying an AC-PC (anterior commissure - posterior commissure) in an image of the patient. In at least some examples, obtaining atlas registration information includes obtaining an image of the patient and registering the image to an atlas. In at least some examples, obtaining atlas registration information includes comparing the patient to a plurality of population groups of the patient population, wherein an atlas is associated with each of the population groups, and selecting the atlas of the population group that is most similar to the patient. In at least some examples, obtaining atlas registration information includes transforming an image of the patient to register the image to the atlas of the population group that is most similar to the patient.

In at least some examples, determining a new target volume includes obtaining user input of at least one clinical effect; obtaining a plurality of estimated volumes of activation (VOA) associated with stimulations of the patient population, which VOAs are associated with the at least one clinical effect; and based on the VOAs, determining the new target volume. In at least some examples, determining the new target volume includes selecting points for the new target volume that belong to a threshold number or percentage of the VOAs. In at least some examples, the method further includes obtaining a rating for each of the VOAs with respect to the at least one clinical effect, wherein determining the new target volume includes selecting points for the new target volume that have a threshold score based on the VOAs containing the point weighted by the rating of the VOA. In at least some examples, the method further includes filtering the VOAs by a time of day when stimulation of the VOA was performed. In at least some examples, the method further includes filtering the VOAs by a medication used by a patient associated with the VOA. In at least some examples, the at least one clinical effect includes a therapeutic effect. In at least some examples, the at least one clinical effect includes a side effect.

Another example is a system including at least one computer processor, the at least one computer processor configured and arranged to perform the following acts: obtaining a target volume to be activated based on a surgical trajectory for implantation of a leadwire; determining stimulation parameter settings that are estimated to provide stimulation in at least a portion of the target volume; analyzing effects of stimulations performed on a plurality of patients of a patient population; based on the analysis, determining a new target volume; and providing the new target volume.

In at least some examples, the at least one computer processor is configured and arranged to perform the following additional act: obtaining atlas registration information regarding the surgical trajectory for implantation of the leadwire. In at least some examples, obtaining atlas registration information includes obtaining an image of the patient and registering the image to an atlas. In at least some examples, obtaining atlas registration information includes comparing the patient to a plurality of population groups of the patient population, wherein an atlas is associated with each of the population groups, and selecting the atlas of the population group that is most similar to the patient. In at least some examples, obtaining atlas registration information includes transforming an image of the patient to register the image to the atlas of the population group that is most similar to the patient.

In at least some examples, determining a new target volume includes obtaining user input of at least one clinical effect; obtaining a plurality of estimated volumes of activation (VOA) associated with stimulations of the patient population, which VOAs are associated with the at least one clinical effect; and based on the VOAs, determining the new target volume. In at least some examples, determining the new target volume includes selecting points for the new target volume that belong to a threshold number or percentage of the VOAs. In at least some examples, the method further includes obtaining a rating for each of the VOAs with respect to the at least one clinical effect, wherein determining the new target volume includes selecting points for the new target volume that have a threshold score based on the VOAs containing the point weighted by the rating of the VOA.

## BRIEF DESCRIPTION OF THE DRAWINGS

Figure 1 illustrates a remote control device according to an example of the present invention.

Figure 2 shows connections between a remote control device and other system components according to an example of the present invention.

Figure 3 shows connections between a remote control device and other system components according to another example of the present invention, aspects of the different illustrated examples being combinable.

Figure 4 is a flowchart that illustrates a computer-implemented method of refining target volume selection, e.g., over time, according to an example embodiment of the present invention.

Figure 5 is an example strength/duration curve plotting different combinations of power and pulse width values estimated to provide equivalent stimulation, according to an example of the present invention.

Figure 6 is a diagram that illustrates a stimulation and analysis cycle by which target volumes and/or stimulation settings can be refined, according to an example embodiment of the present invention.

Figure 7 is a diagram that illustrates a stimulation and analysis cycle with volume sharing, according to an example embodiment of the present invention.

Figure 8 illustrates a data flow including volume sharing, according to an example embodiment of the present invention.

Figure 9 illustrates components usable for representing an anatomical volume, according to an example embodiment of the present invention.

Figure 10 illustrates generation of a patient-specific atlas based on clustering of sub-sets of a patient population based on clinical effects information associated with VOAs of the patient population, according to an example of the present invention.

Figure 11 illustrates an example of a trajectory between a current VOA and an optimal VOA, according to an example embodiment of the present invention.

Figure 12 shows an example of a neighborhood effects map, according to an example embodiment of the present invention.

Figure 13 illustrates a cloud management paradigm with integration of a hospital image system, e.g., picture archiving and communication system (PACS), patient record system including information on patient assessments, a patient programming system, and a patient analytics system, according to an example of the present invention.

Figure 14 illustrates a paradigm in which surgical planning, stimulation programming/planning, and analytics modules are integrated, according to an example of the present invention.

Figure 15 is a further illustration of cloud integration of various modules, according to an example of the present invention.

## DETAILED DESCRIPTION

Certain aspects and embodiments described in the following may not be covered by the wording of claim 1 and are merely provided for illustrative purposes. The scope of the present invention is defined by the claims.

Aspects of the present invention pertain to management of data in a central location for access at multiple locations, by various machines, and via various applications. Aspects of the present invention pertain to an interface of a stimulation setting remote control in a clinical mode. Aspects of the present invention pertain to sharing of target volumes of activation. The applications and systems via which the various data is created and/or accessed and/or used and/or in which the described interfaces may be presented may include any one of those described in U.S. Pat. App. Ser. No. 12/454,330, filed May 15, 2009 ("the '330 application"), U.S. Pat. App. Ser. No. 12/454,312, filed May 15, 2009 ("the '312 application"), U.S. Pat. App. Ser. No. 12/454,340, filed May 15, 2009 ("the '340 application"), U.S. Pat. App. Ser. No. 12/454,343, filed May 15, 2009 ("the '343 application"), U.S. Pat. App. Ser. No. 12/454,314, filed May 15, 2009 ("the '314 application"), U.S. Provisional Pat. App. Ser. No. 61/468,884, filed March 29, 2011 ("the '884 application"), U.S. Provisional Pat. App. Ser. No. 61/468,887, filed March 29, 2011 ("the '887 application"), U.S. Provisional Pat. App. Ser. No. 61/468,891, filed March 29, 2011 ("the '891 application"), U.S. Provisional Pat. App. Ser. No. 61/468,897, filed March 29, 2011 ("the '897 application"), and U.S. Provisional Pat. App. Ser. No. 61/468,901, filed March 29, 2011 ("the '901 application").

Aspects of the present application pertain to subject matter described in U.S. Pat. App. Ser. Nos. 13/571,078 and 13/570,998, the content of both of which were filed August 9, 2012. Aspects of the present application also pertain to subject matter described in U.S. Provisional Patent Application Serial Nos. 61/521,626 filed on August 9, 2011, 61/521,641 filed on August 9, 2011, 61/521,632 filed on August 9, 2011, 61/676,000 filed on July 26, 2012 and 61/676,014 filed on July 26, 2012.

Aspects of the present application also pertain to subject matter described in U.S. Pat. App. Ser. No. 13/431,232 ("the '232 application) filed March 27, 2012, which claims priority to U.S. Prov. Pat App. Ser. Nos. 61/468,884, 61/468,887, 61/468,891, 61/468,897, and 61/468,901 which were all filed March 29, 2011.

**Cloud Data Management**

A Guide software for stimulation therapy may require various data used at various points of time. Example embodiments of the present invention provide for getting the information to go to the right place at the right time. Additionally, example embodiments allow for patient data to travel with the patient. Information may include the graphics, but may also include other non-imagery data, as noted above, such as side effects, tremors (e.g., measured by an accelerometer in the implantable pulse generator (IPG)), medications, and other clinical data that may be updated over time.

In an example embodiment of the present invention, a patient can be assigned and given an identification (ID) card that has the patient's data. For example, the card may be inserted into, or otherwise be provided in communication with, a computer and data on the computer may be recorded on the card. However, as explained in connection with the alternative embodiments below, the data need not be stored on the card, and the card would then only be used for obtaining access to the data, which can be stored remotely, for example, in a central server.

In an alternative example embodiment, the patient has a card that includes a pin number that one can use to access the patient's information.

In an alternative example embodiment, the patient has a card that includes an account number that, when input to a computer along with a pin number from the user's memory, allows access to the patient's information.

In an alternative example embodiment, the card includes a number that, when the card is swiped in a device connected to a computer, is read by the computer, and that thereby allows access to the data of the patient associated with the number. In an example embodiment, the number can be one on the patient's implanted IPG, which is already managed to have unique numbers. Use of such a number, rather than patient name, can help with anonymization issues. That is, the data is stored in connection with a number, rather than in association with data by which can identify the patient.

A problem is where data is recorded before the user obtains the IPG. In this instance, a temporary number may be assigned, and then, when the IPG is implanted, a switchover may be made to the number of the IPG.

Use of the IPG number may be advantageous because even if the user loses the card, the user can obtain the information by using the IPG which obviously does not get lost.

In an example embodiment of the present information the IPG may transmit a signal with the number. According to this embodiment, the card may be omitted. Instead, the signal of the IPG indicating the unique number (or any unique signal that may be omitted by the IPG which unique signal is associated with the particular patient) may allow the data access. Not only the IPG, but any RFID device provided to the user can be used to provide the number. However, it is preferred to use a device implanted in the patient, so that it is ensured not to be lost.

The computer may "talk" to the signaling device on the patient to get the number needed to obtain (or send to the server) the information. For example, the user may connect a device to a port of a computer (e.g., a USB port, a serial port or any other communications port), which device is configured to receive a signal from the IPG, which indicates some number. The number is sent to the central server and then the information is obtained. This can be beneficial for security purposes as well because it allows the system to be controlled not to send the information unless the device recognizes that the patient with the correct IPG is physically present at the computer terminal at which the information is being requested or via which information is being stored.

The guide system, including the visualization package, the clinician programmer (CP) by which the clinician actually programs the IPG, or the patient programmer system by which the IPG settings can be changed without the clinician, and the analytic system (described below) can each access the information in the cloud. In an example embodiment, any web browser may be used to access the information.

Different users can have different types of access. For example, a patient may have a certain level of access, a health care provider may have another level of access, and a relative (who is not the health care provider) can have yet another level of access.

In an example embodiment, the IPG may be programmed to generate, based on a user-input code, a second code that provides a specific level of access. The second code may be unique to the user (e.g., a particular health care provider). In this manner, different levels of access may be provided to data generated using the same IPG. Thus, for example, a health care provider may only be able to access IPG data generated at the provider's facility, but may not have access to IPG data generated at other provider facilities, even though the IPG is the same.

In an example embodiment, health care providers may be provided with the ability to access the data without the patient (and therefore the IPG) being physically present. For example, the provider may store the patient's access credentials (e.g., IPG number) the first time the patient visits, so that patient data can be obtained prior to the next visit.

Additional data that can be included in the central location for access as described above is analytics information as described below and the data used for the analytics.

Additional information stored in the cloud can be a log of the changes entered by the patients to their parameters using the remote control, which changes could be time-stamped, as described below. The remote control will record the information. The remote control can be plugged into a computer which then sends it to the central location for storage in association with the number that is unique to the patient. Alternatively, it can be sent continuously or periodically through, e.g., a wireless connection, e.g., via a cellular connection. Alternatively, the remote control can connect to a device through a wireless connection, and, when so connected, it can send the information to the central server.

Additionally, the detailed computations - fusion of MR/CT, fusion of atlas to MR, lead location, VOA generation, etc. (see all computations and data described in the'330, '312, '340, '343, '314, '884, '887, '891, '897, and '901 applications) - can be performed at a server and the resulting information can be stored centrally.

In an example embodiment, transformation matrix parameters can be stored centrally. For example, the central server, the IPG, and/or the remote used by the patient would store the transformation matrices, such as how to transform the atlas to the patient's MR. Additionally, it could store the location of the lead in the resulting patient-specific atlas. It could also store an atlas identifier to identify which atlas is the one being transformed, in case different atlases are used at different times, e.g., because of a software upgrade.

In an example embodiment of the present invention, information from sensors monitoring a patient condition, for example, while stimulation is being performed, immediately thereafter, or during a longer period spanning a stimulation therapy including periods of on and off stimulation states, is uploaded to a cloud data store which is accessible by clinicians, doctors, and/or the patient logging into various stimulation control modules, e.g., a stimulation programming and/or planning module. According to an example embodiment, the sensor information is associated with VOAs as further described below which is used for patient population analytics, as further described below.

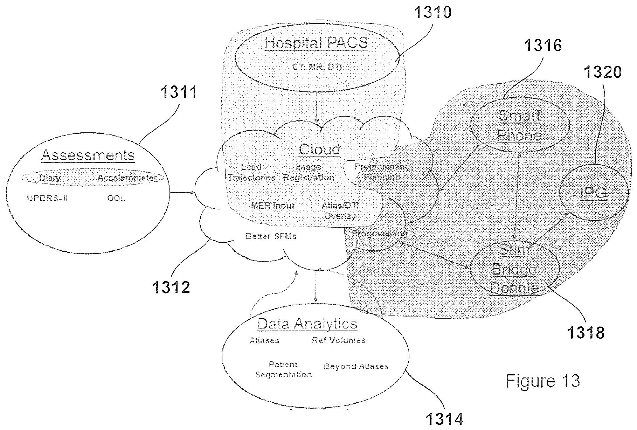

Figure 13 illustrates a cloud management paradigm with integration of a hospital image system, e.g., picture archiving and communication system (PACS) 1310, patient record system including information on patient assessments 1311, a patient programming system, and a patient analytics system. Patient records, including assessments are uploadable to a central cloud data store 1312. Patient images are also uploadable from the PACS system to the cloud data store. Data analytics 1314 as described below are also uploadable to the cloud 1312. The cloud may include a server which maintains programs accessible from terminals, e.g., over the Internet, for determining lead trajectories for surgical planning based on the patient images and patient assessment information. The programs can also include ones usable for registering a lead model to a patient atlas, e.g., after the lead has been implanted. The cloud can further include programs used for planning stimulation programs, and for actually programming the implanted pulse generator (IPG). A smart phone 1316 can be used to access these programs. A stimulation bridge 1318, which can be embodied in a dongle, can interface between the IPG 1320 and the stimulation programming module for programming the IPG to cause the implanted leadwire to generate electrical pulses according to the program. Information concerning the effects of the stimulation can be obtained automatically and/or manually input, e.g., via a patient interface module, which can also be accessible from the cloud to update the analytics information. The effects information are correlated with the stimulation program set in the programming module and for which the program planning module generates estimated VOAs.

## Data Export/Import

According to an example embodiment of the present invention, a same stimulation-related and/or anatomical atlas (patient-specific or non-patient-specific) data can be accessed via multiple computer terminals. For example, in an example embodiment, such data is saved to a file stored at a central location accessible from multiple computer terminals.

In an example embodiment of the present invention, such data can be shared with other users, e.g., as an attachment to a communication (e.g., an e-mail) or by providing the other user access to the centrally stored file. Such data often includes information that is specific to a patient and private. Accordingly, in an example embodiment of the present invention, the system includes an anonymization feature for stripping from the shared data any data that identifies the patients. For example, in an example embodiment, the system includes a soft or hard selectable button, in response to selection of which the system strips private data. For example, the system can be programmed to remove patient name, address, social security, etc. In an example embodiment, in response to selection of the anonymization button, the system outputs a list of types of data which the user can select to strip. In an alternative example embodiment, the system saves the information centrally with all of the data, and depending on permissions set for a user attempting to access the centrally stored data, either provides the data with the private information or provides a stripped-down version of the data.

According to an example embodiment of the present invention, the system is configured to transmit VOAs or other volumes in the form of centroids (e.g., center of mass of the volume), e.g., in combination with other elliptically based information, as further described below. In an example embodiment, volumes can be transmitted / opened in a CAD format. Volumes can be exported to other users in the reference frame of the patient for whom the volume was generated. Alternatively, the volume can be transformed to a common reference frame, e.g., a common atlas, and transmitted to other users in this generic form. In an example embodiment, the system is configured to export the VOA or other volume as a full 3D mesh of the volume.

In an example embodiment, the system is configured to export geometric primitives of the volumes, e.g., of the VOAs. For example, in an example embodiment, the system saves / transmits the parameters of a sphere or ellipse that best matches the VOA, which allows for the amount of data that is to be saved and/or exported to be scaled down. Such data can be sent, for example, in an Excel format, as a comma delimited file, as a text file, or as a CAD file. Providing the volume information in such forms can be beneficial to allow one to use third party applications not adapted to interpret/process the more intricate volume data the Guide software is configured to process.

For example, a volume, e.g., a VOA, can be saved/transmitted as a set of points in 3D space with information on how those points are connected. Alternatively, the volume can be saved as a combination of a centroid and, for example, a radius. The radius can be that which is determined to provide an ellipse that optimally overlaps the volume, e.g., with least combined difference of overlap and underlap to the volume or the smallest ellipse that covers all points of the volume. For example, the system can store a plurality of volumes, each as a row including the data {x, y, z, r}, where x,y,z represent a point in three-dimensional space at a center of mass of the volume and r represents the radius.

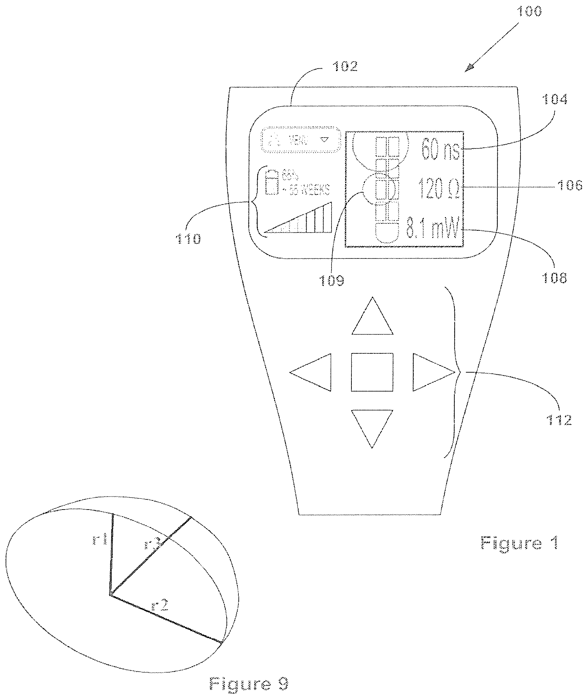

According to a variant of this embodiment, the system can include multiple radii to represent the volume, for example, to represent a three-dimensional ellipsoid. For example, three radii r1, r2, r3 can be used, each for a respective one of the x, y, and z directions, as shown in figure 9. In an example embodiment of the present invention, the system further provides additional data indicating an orientation of the ellipse in an atlas or anatomical image space. This is because the same ellipse can be orientated in a number of ways relative to the same anatomical space. In an example embodiment of the present invention, the orientation information is provided as any two of three angles. For example, a first angle can represent an angular offset from the superior-inferior line, a second angle can represent an offset from the anterior-posterior line, and a third angle can represent an offset from the medial-lateral line. Thus, each of the radii can be a radius drawn along one of those lines, and each angle can be a respective offset for a respective one of those radii.

Alternatively, geometric primitives other than radii and/or x,y,z coordinates can be used as an estimate of the volume. For example, a geometric primitive to be used for characterizing a sphere can be a diameter; geometric primitives to be used for characterizing an ellipsoid can be axes lengths; geometric primitives to be used for characterizing a hexagon can be side lengths; geometric primitives to be used for characterizing a pyramid can be a height, lower radius, upper radius, etc.

Other data can be provided to further define the volumes, e.g., warping parameters, such as an indication of an amount of warp, a direction of warp, etc.

In an example embodiment, a geometric volume can be represented by identification of elements, voxels, or nodes that are included or excluded, e.g., the system includes a standard format by which to present such information.

In an example embodiment of the present invention, a more precise volume can be saved / transmitted in an Excel, comma-delimited, or other similar format. For example, in an example embodiment, a volume can be represented using a first record corresponding to a volume includes a plurality of tuples, each tuple corresponding to a single point in two or three dimensional space, and a second record identifying each pair or triple of connected ones of the points. For example, the following record can be stored $\{x_1,y_1,z_1;x_2,y_2,z_2,x_3,y_3,z_3; \ldots\}$, where each x,y,z, combination is one point on a perimeter of the volume. The following additional record can be stored $\{1,2,3;3,4,5; \ldots\}$, where each combination of numbers identifies a respective combination of tuples that are connected. For example, "1,2,3" indicates that the point of tuple 1 is connected to the point of tuple 2 and to the point of tuple 3. If points of a two-dimensional volume are stored/transmitted, the second record may be in the form of, for example, $\{1,2;2,3;3,4; \ldots\}$, where each combination of numbers identifies a respective combination of tuples that are connected. For example, "1,2" indicates that the point of tuple 1 is connected to the point of tuple 2, etc.

## Integration of Surgical Planning and Stimulation Programming Systems

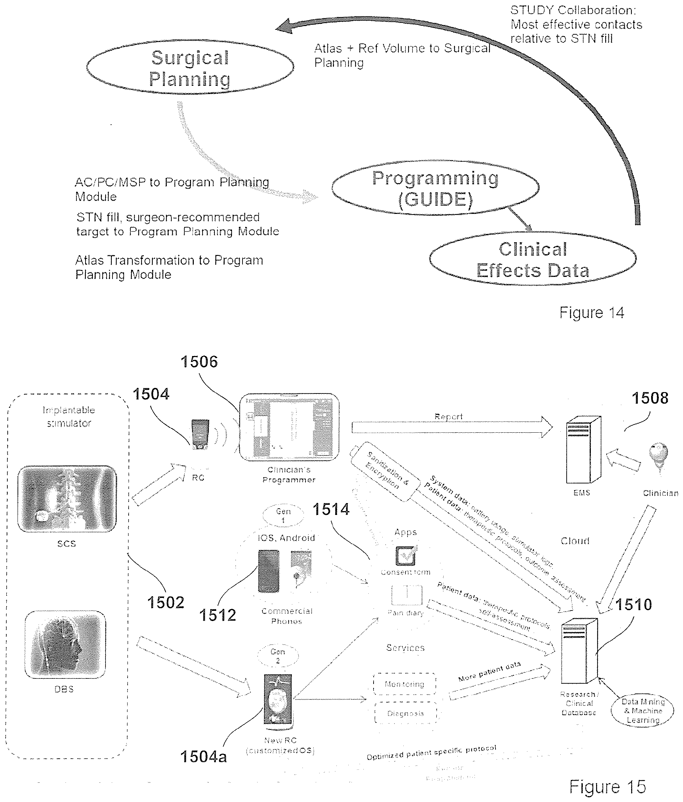

Figure 14 illustrates a paradigm in which surgical planning, stimulation programming/planning, and analytics modules are integrated, e.g., by direct data export from one module to another or by saving of information in a cloud which the information is accessible by all of the modules. For example, the AC/PC/MSP (anterior commissure/posterior commissure/median sagittal plane) can be identified in a surgical planning system, and can be accessed by the programming module to program stimulation settings based on the identified anatomical markers. Similarly, a surgeon can select a target structure or other target volume, e.g., manually drawn, as a target for stimulation, which the program planning module obtains for use for finding stimulation parameters that are best for providing stimulation of the target region. Similarly an atlas registration can be performed using the surgical module, and the registered atlas can then be used with the program planning module to accurately select stimulation parameters. In an example embodiment, for the atlas registration, the surgical planning system transmits an identification of a location of a relevant MR, an identification of a relevant CT, and a number matrix for transformation of the MR and CT information to yield patient atlas.

Pre-op lead location information and a pre-op CT can be provided to the stimulation programming module for use to determine the lead location, on which basis to select stimulation settings for stimulating a target volume. MER data of the surgical planning module can also be accessed by the programming module.

The programming modules can be used for selecting stimulation programs, and generating estimated volumes of activation. Further, the programming module can obtain clinical effects data associated with the VOAs corresponding to the implemented stimulation programs, on which basis analytics are performed, as described below. The analytics are used to refine target areas, and the information can be provided back to the surgical planning modules, for view therein by a user, e.g., a surgeon, to better select a target region for future surgeries.

Though not shown in Figure 13, the surgical planning system of Figure 14 can have access to the cloud shown in Figure 13 to facilitate the described system integration.

Figure 15 is a further illustration of cloud integration of various modules. A system can have any combination of the following components: an implantable stimulator 1502 (including, for example, a IPG and one or more leads); a remote control (RC) 1504, 1504a; a clinician programmer (CP) 1506, cloud or other central storage 1508 with a database 1510; and a smartphone 1512 or other computing devices that can access applications 1514 (Apps). Figure 15 illustrates representative flows of data, information, and communication between these components including examples of the types of information that may flow. It will be understood that information flow, although exemplified in one direction in Figure 15, can often flow in both directions between system elements.

A web-based platform has at least three advantages over a local device paradigm. First, fixed hardware platforms inevitably become obsolete as hardware changes occur. Such a scenario would not occur in a web-based platform. Second, a web-based platform would enable separation of client and server and allow deployment of algorithms to which terminals can gain access over time. Third, a web-based platform could dramatically aid online analysis.

The modules can include a Patient Data module, a Registration module, and a Patient Programming module. The Patient Data module allows the user to browse and select patient datasets. The Registration module then allows the user to select a pre-operative MRI dataset and a post-operative CT dataset. The user then can fuse these datasets and view the fused datasets either individually or in an overlay. In addition to fusing MR and CT datasets, the Registration module allows the user to automatically or manually determine the positions of stimulation leads from the post-operative CT images and also enable semi-automatic registration of the patient's MRI on to a standard Atlas. Once the lead positions are determined and registration is completed, the Patient Programming module allows the user to visualize areas of the brain activated in response to different DBS stimulation settings. Since these areas are depicted in the software in a reference frame that includes relevant atlas structures, the user can utilize the display as a guide for optimal choice of stimulation parameters.

These functionalities can be provided in a web-based system, which can include the following components: (1) a web-based front end through which the user will control the program, (2) a back-end server which will perform a significant portion of the computations required by the user, and (3) a back-end database to serve as a Laboratory Information Management System (LIMS). In an example embodiment, the front-end runs in at least one browser and has a secure connection to the back-end server.

### *Front End:*

#### Patient Programming Module:

The Patient Programming Module allows the user to select DBS stimulation settings and depicts the corresponding VOA. In an example embodiment, the computations for generating the VOA are performed on the client. Alternatively, they are performed on the server with 3D models being pushed to the client. Either way, the system outputs VOAs and atlas structures for varying DBS settings. The system rotates Atlas and VOAs through (at least) a discrete set of angles to enable appreciation of intersections between the VOAs and atlas structures and changes of VOAs with varying parameters.

#### Registration Module:

This module allows scrolling through MR and CT datasets, fusion of MR and CT, auto-detection of leads from CT, and registration of MRI onto atlas. With respect to the registration, in an example embodiment, the AC-PC (anterior commissure-posterior commissure) is selected and an atlas is selected to match the atlas AP-PC to patient AC-PC. In an alternative example embodiment, there is a fully automated registration algorithm, e.g., using B-splines. In an alternative example embodiment, the system maintains a large database of patient population MRs, and the system finds the best match between the current patient's 3T MR and the database of 7T MRs (or the best few matches).

#### Patient Data Module:

This module allows users to see which datasets are available on the server. The user is able to select one pre-operative MR dataset and one post-operative CT dataset for further analysis. If the LIMS exists, patients can be selected based on their behavioral scores or any other criteria of choice. Users can also view behavioral measures, such as UPDRS scores as bar plots, and accelerometer readings as time series/power spectra.

### *Back End:*

In an example embodiment, the back end has the ability to accept secure requests from the client and display available datasets, provide feedback slices of MR and CT when the user scrubs through the data, perform fusion when user selects one CT and one MR dataset from available list and to feedback fused MR and CT datasets, perform registration and feedback registered MR+CT and atlas structures to the client, and perform VOA calculation and feed the results back to the client.

### Data Capture on the Remote Control (Patient Programmer)

A need in neuromodulation is to have some way to blind a patient as to whether the patient's device is on or off. This is helpful, for example, for clinical trials. This is difficult because the patient usually has a remote control that informs the patient of this information. Therefore, according to an example embodiment of the present invention, the remote control is provided with a clinical mode, where the remote creates the illusion as though the device is on, e.g., the user can interact with a user interface to raise or lower the stimulation amplitude and/or other settings, when really nothing is happening in response, although the remote gives the appearance as though the system is responding to the user's commands.

For example, referring to figure 1, a remote control 100 can include a display screen 102 including graphical information regarding parameters set in the IPG, including, for example, the pulse width 104, current amplitude 106, and power amplitude 108 of electrodes of an implanted leadwire controlled by the IPG. The display screen 102 can include additional information regarding the IPG, such as one or more representations of its battery power and life 110. The remote control 100 can include one or more buttons 112 via which the patient can input instructions to the IPG for modifying one or more of the settings. For example, the user can, in an example embodiment, select an electrode and input a desired amplitude setting, polarity, etc. for the selected electrode, e.g., by textual input, or by selecting an up or down arrow to raise or lower a setting. In an example embodiment, a graphical representation of an electric field 109 drawn about one or more graphical representations of respective electrodes can be shifted, e.g., using arrow keys, which is interpreted as an instruction to modify one or more settings to provide the shifted electric field. The remote control 100 can include other features for input of stimulation settings, for example, as described in the '330, '312, '340, '343, and '314 applications.

In an example embodiment, when the remote control 100 is in the clinical mode, if the user manipulates the input elements, e.g., buttons 112, of the remote control 100 to modify the settings of the IPG, the remote control 100 updates the graphical user interface (GUI) to reflect the input modifications, without the input modification instruction being implemented at the IPG. For example, the remote control 100 can refrain from responsively transmitting modification instructions to the IPG, or the remote control 100 can transmit the instruction, but the IPG can ignore the instruction. According to the latter embodiment, the IPG enters the clinical mode, while the remote control 100 is blind to whether the system is in a clinical or a regular mode. Although the instruction to modify the settings is not executed, the remote control 100 can display the modified setting, such as a modified pulse width, current amplitude, and/or power setting, and/or the location(s) of one or more of the displayed current fields, as though it had been implemented.

The remote control 100 can also modify the battery power and life representations 110 to reflect the modifications as though they had been implemented. For example, in response to instructions for modifications that when implemented would cause a change in the battery power and expected battery life, the remote control 100 may update the battery power and life representations to reflect such change, although the modifications are not implemented.

Additionally, in an example embodiment, where the IPG does not perform with the leadwire a stimulation, such that battery power of the IPG is not being used or is used at a very low rate, but the patient is led to believe in clinical mode that a stimulation program is being applied, the remote control 100 modifies the battery power and life representations 110 as though the stimulation program is being applied.

In an example embodiment in the clinical mode, the remote control 100 provides an output, e.g., a graphical, audible, or tactile output, warning of low battery power of the IPG, such that a recharge is suggested, in accordance with the indicated stimulation program, although the program is not being applied and the battery power is in fact not depleted.

In an example embodiment, the remote control 100 includes a charger for charging the IPG battery. For example, the remote control 100 can include a wire with a coil for inductively charging the IPG. In the clinical mode, where the IPG battery is shown to be at less than its actual charge level, and the user uses the remote control 100 in order to charge the IPG battery (although the battery might be fully charged), the remote control 100 may update the battery power and life representations 110 to reflect an increase in the battery power and remaining life, as though being increased from the low battery power and life indications.

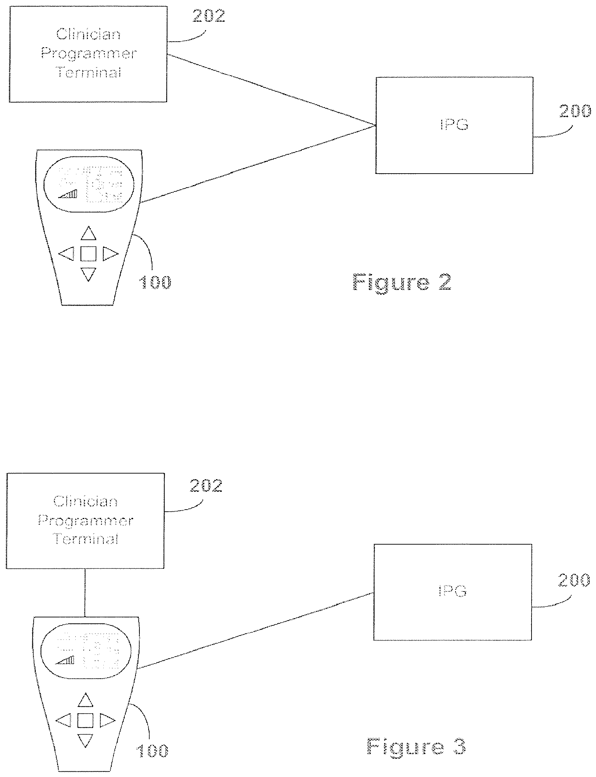

In an example embodiment as shown in figure 2, the remote control 100 communicates with the IPG 200 and also interacts with a CP (clinician programmer) terminal 202, e.g., a laptop or other computer terminal in which a clinician programmer application is executed.

For example, a user operating the CP terminal 202 can input upper and/or lower bounds of a parameter of the IPG 200, e.g., upper and lower amplitudes for a stimulation program. The CP terminal 202 can transmit, e.g., wirelessly, data to the IPG 200 for setting the upper and/or lower bounds within the IPG 200. The patient can operate the remote control 100 to set one or more stimulation parameters of the IPG 200. However, the IPG 200 ignores parameter settings received from the remote control 100 that are not within the upper and/or lower bounds set by the CP terminal 202. Alternatively or additionally, in an example embodiment, when the remote control 100 communicates with the IPG 200, the IPG 200 responsively notifies the remote control 100 of the restrictions set by the CP terminal 202, and the remote control 100 thereafter refrains from transmitting to the IPG 200 settings that do not comply with the restrictions, until the IPG 200 informs the remote control 100 of removal or modification of the restrictions.

In an alternative example embodiment, as shown in figure 3, the CP terminal 202 communicates restrictions concerning the stimulation settings to the remote control 100, and the remote control 100 refrains from transmitting instructions to the IPG 200 that do not satisfy those restrictions. For example, if the user inputs an instruction to set a stimulation amplitude to a level that is higher than an upper bound communicated by the CP terminal 202 to the remote control 100, the remote control 100 ignores the user input instruction, e.g., at least with respect to responsively transmitting an instruction to the IPG 200.

In an example embodiment, such bounds may be set in a clinical mode, such that the remote control 100 responds to a user input to modify a parameter to a level that is beyond that which is allowed by the instructions of the CP terminal 202 by accordingly modifying the GUI in the display screen 102, but refrains from sending instructions to the IPG 200 to set a parameter to the impermissible level, or, according to the embodiment described with respect to figure 2, the remote control 100 possibly sends the instructions, which the IPG 200 ignores according to the restrictions received from the CP terminal 202. However, as noted above, in an example embodiment, the IPG 200 can also communicate to the remote control 100 the restrictions set by the CP terminal 200. Additionally, in an example embodiment, the IPG 200 informs the remote control 100 that it is operating in clinical mode, and the remote control 100 responsively modifies its output as described above to provide the illusion of changes being implemented even though they are not being implemented.

The embodiment described with respect to figure 2 may be preferable over the embodiment described with respect to figure 3 so that if a new remote control is used, the CP terminal 202 need not resend the restrictions to the new remote control.

In an example embodiment, a clinician can put the device in a mode such that it goes on and off at various preset times, and the patient does not know when it is on or off. For example, the CP terminal 202 can send instructions concerning such a stimulation program to the IPG 200, which can, in turn, inform the remote control 100 of the stimulation program, e.g., which cannot be overridden by the patient via the remote control 100, or which can be overridden by the patient in only defined limited ways, e.g., for safety reasons. Meanwhile, the remote is configured to receive from the patient input indicating how the patient is doing. Such feedback may include input of a number on a predetermined scale. For example, the device outputs a reminder to input the information. So now clinical trial data can be obtained where information on how the patient is doing is periodically received, and the patient does not know when the device is on or off. Patient feedback may be time-stamped for subsequent clinical analysis.

Additionally, in an example embodiment, the device changes the program used to stimulate at various intervals, and the remote does not indicate to the patient which stimulation parameters are being used at that time. The patient then records over time how the patient is doing. Over time, the device learns which program is best for the patient by determining for which parameters the patient has been indicating the patient feels best. For example, the device can iterate through a number of settings for each electrode, gradually increasing the amplitude at a respective electrode contact of the leadwire, and continuously do so as long as the patient provides good feedback about that setting. In this example embodiment, the patient does have the ability to manually override the predetermined settings, for example, in case the device automatically sets a dangerous setting. Therefore, recorded feedback may include patient override requests.

In an example embodiment, there can be sensors that sense how the patient is doing. In addition to the patient manually entering how the patient is doing, the sensor information can be used to indicate how the patient is doing. Such sensors may include, for example accelerometers or other sensors that detect motor skill and/or cognitive functioning, for example, tremor (motor skill), dwell times (motor skill / cognitive), etc. The sensors may be integrated into the remote, the IPG or any other hardware that the patient carries.

The information on how the patient is doing and the related stimulation parameters can be stored at the central server.

In an example embodiment the preset stimulation program, whether including a single steady set of parameter settings, or including a plurality of sets of parameter settings that are implemented at different times, e.g., at different intervals, can be set in a clinical mode, as described above, where the GUI of the remote control 100 is modified to reflect changes entered by the patient, although such changes are not implemented. In an example embodiment, even in a clinical mode, the system may allow the user to override certain settings for safety reasons.

According to an example embodiment the system may be configured to perform a clinical study for testing various settings, including, for example, testing a response to an on-low setting at which a low power stimulation is applied, an on-high setting at which a high power stimulation is applied, and an off setting at which no stimulation is applied. In an example embodiment the clinical stimulation program includes cycling through the three (or more) settings one or more times at equal or varying intervals. As explained above, the patient can be blind to the changes, and the system can be configured to record information regarding the patient's condition at various points during the stimulation program, which information can be obtained from user input and/or from sensors.

In an example embodiment of the present invention, the system may include an electronic diary ("e-diary") feature for recording a log of time-stamped patient condition information, and for recording time-stamped information concerning the stimulation settings, so that the patient condition information can be associated with particular stimulation settings. Certain of the recorded information can pertain to factors that are not a result of the stimulation settings, e.g., which medication(s), if any, the patient is taking. Other of the patient condition information can be associated with a combination of the stimulation settings and the medication(s) the patient was taking at the time associated with the patient condition information.

In an example embodiment of the present invention, for obtaining patient condition information, medication information, etc., the remote control 100 includes user input hardware and/or software controls via which the patient can enter information. In an example embodiment, the remote control is configured to receive input from the patient of entry of a number on some number scale, e.g., 1-10, of how the patient is feeling. In an example embodiment of the present invention, the remote control 100 includes a "good" button and/or a "bad" button by which the patient can generally indicate whether the patient generally feels good and/or bad. In an example embodiment of the present invention, the remote 100 includes soft and/or hard buttons (or check boxes, radio buttons, etc.) for predetermined significant events, such as, for example, falls, seizures, etc., which the patient can select when such an event occurs. The system can record a time-stamped entry in the e-diary noting the occurrence of the event indicated by the selection of the corresponding event input.

In an example embodiment, the remote control 100 stores the e-diary information locally in a long-term storage of a memory device of the remote control 100. In an example embodiment, the remote control 100 alternatively or additionally transmits the e-diary information to the IPG for storage therein. In an example embodiment of the present invention, the e-diary information is alternatively or additionally uploaded to a central server, e.g., as discussed above under the "Cloud Data Management" section.

As noted above, in an example embodiment the system is configured to record a time-stamped log of the stimulation settings. In an example embodiment, the IPG records time-stamped stimulation settings at predetermined intervals. In an alternative example embodiment, the IPG records time-stamped stimulation settings responsive to a change to the stimulation settings. In an example embodiment, after recording initial settings, subsequent settings are recorded as a change to the immediately preceding settings.

In an example embodiment of the present invention, the system correlates respective portions of the patient-condition information to respective settings based on the timestamps, and automatically modifies settings based on the correlation. For example, in an example embodiment, the system detects a trend, e.g., that with increase of a certain parameter between a first time and second time, the patient condition has deteriorated, and therefore modifies the settings, e.g., in a reverse direction in response to a detected deteriorated condition and/or further in a same direction in response to a detected improvement in condition.

In an alternative example embodiment, or additionally, the system outputs a report of the effects of the settings on the patient condition. For example, in an example embodiment, the system outputs a report that an increase or decrease of parameter 'x' has been detected to be associated with a deterioration or improvement of condition 'y'.

In an alternative example embodiment, or additionally, the system outputs a timeline covering a time period including some or all of the time-stamped times and further outputs against the timeline (a) a graph representing changes to one or more patient conditions indicated by the patient-condition information, and (b) identifications of the settings prevailing at different times of the timeline.

**VOA Selection for Target Volume**

A target volume can be selected, e.g., by a user or by the system, e.g., based on clinician input, the patient's information (such as a patient disorder, patient history, etc.) population information (such as learned information from one or more other patients), therapeutic goal, etc. In an example embodiment of the present invention, the system outputs suggested stimulation settings and/or outputs a graphical VOA corresponding to suggested stimulation settings for such a target volume.

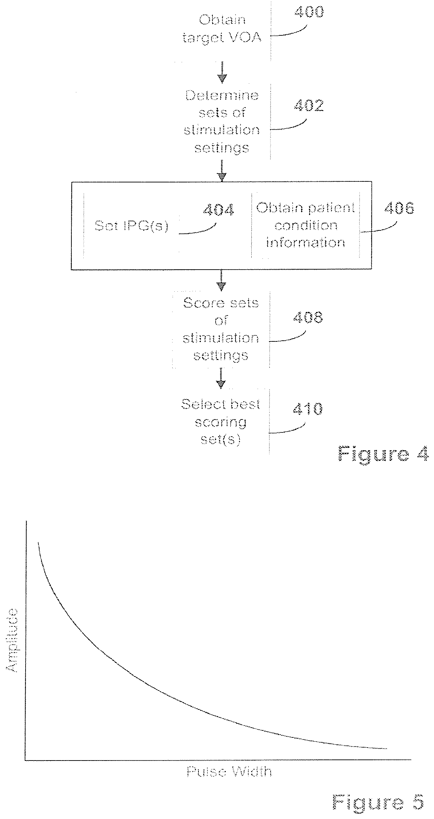

Referring to figure 4, at step 400 the system obtains a target volume. At step 402, the system determines a plurality of sets of stimulation settings that provide VOAs considered similar to the target volume. The system can perform such a determination according to a predetermined one or more conditions, such as a degree to which a VOA must overlap the target volume and/or a maximum amount by which the VOA can spill beyond the boundaries of the target volume. In at least some embodiments, the conditions can be selected and values chosen by the clinician or other user. At step 404, the system sets the IPG of one or more patients, e.g., for whom the target volume (or a similar target volume) is obtained, to test some, or each, of the plurality of sets of stimulation settings. The settings may be tested on a single patient, e.g., the particular patient for whom the system will output the suggested optimal settings in view of the obtained target volume, or across a patient population. According to an example embodiment, where the settings are tested across a patient population, different ones of the sets of stimulation settings can be tested in parallel. According to an example embodiment, where the settings are tested by applying the sets of stimulation settings to a single patient, the system cycles through a program in which different sets of the stimulation settings are applied in a sequence over time, different ones of the sets being applied during different intervals of the program.

At step 406, the system obtains patient condition information for the tested settings. Steps 404 and 406 may be performed concurrently. That is, while a patient is stimulated by a set of stimulation settings, the system is configured to obtain information concerning the patient's condition, e.g., via patient input or by sensor signals, as described above. The information and settings can be time-stamped. The condition can include therapeutic effects, side effects, patient ratings, and the like, or any combination thereof.

In an example embodiment, for each of the patients on which the sets of stimulation settings are tested, the system associates the patient condition information with the set of stimulation settings applied to the respective patient for whom the respective patient condition information was obtained, the association being based on a determined correspondence of the timestamps of the patient condition information and the applied set of stimulation settings, as explained above.

In an example embodiment, at step 408, the system assigns a score to each of the sets of stimulation settings based on the patient condition information associated with the respective set. For example, different weights can be applied to different types of patient condition information to calculate an overall score. According to an embodiment in which the sets of stimulation settings are tested across a patient population, the system can test a same one of the sets on multiple patients. In an example embodiment, the system calculates an average score of the scores calculated for each patient for whom the set of stimulation setting was applied.

At step 410, the system may compare the calculated scores and select a predetermined number or percentage of the best scoring, e.g., the 3 highest scoring or the single highest scoring, tested set(s) of parameter settings as candidate parameter settings (and associated VOAs) to output as suggestions for a patient for whom the same or similar target volume is selected.

The target volume selection and/or the selection of suggested settings can be performed on any computing device, e.g., a CP terminal.

In an example embodiment the sets of settings can be tested in a clinical mode during which the patient is blinded to the settings.

**Programming Based on IPG Efficiency:**

It is possible for a plurality of different sets of stimulation settings to result in the same or similar VOAs, where certain ones of the sets of stimulation settings are more electrically efficient than others of the sets. For example, similar tissue activations may be obtained by varying the electrical amplitude and pulse width. For example, first settings have a high amplitude and a short pulse width can be equivalent or approximately equivalent to second settings having a lower amplitude but a longer pulse width. For example, equivalence can be measured in terms of power or total current delivered or by any other relevant measure, including the strength/duration curve discussed below, and "approximately equivalent" can be, for example, a variation of 1, 2, 5, or 10 percent.

In an example embodiment of the present invention, the system can in step 402 select a plurality of electrically equivalent settings that differ in their respective amplitudes and pulse widths. It is noted that although such sets of settings may be considered electrically equivalent and/or calculated to produce equivalent or substantially equivalent VOAs, it may nevertheless occur that the different sets of settings produce different clinical effects. Therefore, in an example embodiment, the system tests these equivalent sets at steps 404 et seq.

In an example embodiment of the present invention, the system also assigns a weight to electrical efficiency for the calculation of the scores at step 408.

In an example embodiment of the present invention, the system finds a plurality of sets of electrically equivalent stimulation settings using a strength/duration curve. For example, in an example embodiment, at step 402, the system determines a set of stimulation settings that is estimated to produce a VOA that best fits the target volume, and then finds other sets of stimulation settings that are electrically equivalent to the determined set of settings based on the strength/duration curve.

According to an example embodiment of the present invention, the system uses a strength/duration curve that relates to the discharge of an IPG. In an example embodiment, a device is programmed, e.g., automatically, to use the least amount of energy to fill the target volumes based on the strength/duration curve. In an example embodiment, the efficiency of the settings is one of a plurality of factors contributing to a score on whose basis a set of settings is selected, as described above, where, all else being equal, greater efficiency results in a higher score.

In this regard, according to an example embodiment, programming settings are automatically adjusted towards a target volume specified by a user (or otherwise selected). The visualization system is configured to, based on the specified target volume, test settings that use the lowest power consumption while reaching the specified target volume. In an example embodiment, the system also tests settings that yield volumes that approximate the target volume (e.g., slightly larger or smaller than the target volume). Additionally, in an example embodiment, the testing of such settings can be performed during the clinical mode, so that the optimal settings (in terms of a combination of therapeutic effectiveness and power consumption) is obtainable with the aid of feedback from a blinded user.

Figure 5 shows an example strength/duration curve, where the ordinate represents the amplitude and the abscissa represents the pulse width, where the plotted points are all estimated to produce substantially equivalent volumes of activation. The graph shows that the higher the amplitude, the shorter the required pulse width for producing substantially the same volume of activation. That is, a fiber is expected to fire based on a combination of amplitude and duration of the stimulation at that amplitude, so that the higher the amplitude, the shorter the required duration for causing the fiber to fire.

According to an example embodiment of the present invention, for a selected VOA, the system plots the strength/duration curve of values that are estimated to produce the selected VOA, and selects the most efficient of the pairs of values, e.g., assuming all other factors being equal (as noted above, other factors may result in selection of a set of values different than those of the most efficient pair).

For example, the system can obtain a target volume, e.g., user-selected or automatically selected. The system then determines one or more closely matching producible VOAs. The system is configured to select, for each of one or more of such VOAs, a most energy efficient pair of amplitude and pulse width values to produce the respective VOA. For example, where a remaining battery power is 3.7v and a required amplitude is more than 3.6 volts, the system can use capacitors to double the voltage to 7.4v. If less than 7.4v is required, the system can burn off the difference between 7.4v and the required voltage. For example, if 4v is required, the system can burn off 3.4v, which is inefficient. Accordingly, for the 4v requirement, the system can determine from the strength/duration curve whether there is a more efficient pair of settings. For example, the system might determine that the same VOA is producible with a longer pulse width at 3.5v.

## SFM Analytics

Example embodiments of the present invention pertain to determining a target volume by analysis of VOAs for a plurality of patients.

According to an example embodiment, a system can include program code for providing visualization features to output graphical representations of estimated VOAs, e.g., about a representation or image of an implanted leadwire and/or overlaying a model or image of a patient anatomy. The system can include code by which user, e.g., clinicians or patients, can test various settings estimated to produce such VOAs. The system can also include analytics code for determining a target volume, e.g., based on such VOAs tested by the users.

In an example embodiment, the system includes features for facilitating the sharing by users of target volumes the different users have selected. In an example embodiment, the system includes features by which users can share data, such shared data being subjected by the system to analytics to determine optimal target volumes and/or optimal stimulation settings and corresponding VOAs.

## Systems configurations:

In an example embodiment of the present invention, the SFM analytics features are provided as part of a stand alone visualization system. For example, A Guide system can be used to modify/test parameter settings corresponding to VOAs and/or side effect volumes (volumes where stimulation is preferably avoided), and/or to set target volumes, which VOAs, side effect volumes, and/or target volumes can be transported to a separate analytics system on which analyses can be run. In an alternative example embodiment of the present invention, the SFM analytics features are provided in a Guide system including the clinician programmer features by which to program the settings of the IPG, such that the analytics by which to find optimal settings is conveniently on a system used to actually program the implanted device.

According to an example embodiment of the present invention, analytics information and input for performing analytics can be stored remotely via a cloud / internet-based system. In an example embodiment, the data from many systems, e.g., workstations operated by many users, can be stored centrally, and the centrally stored data and/or the resulting analytics information can be accessed by a plurality of users of networked systems.

In an example embodiment, the analytics features are provided in a software package that users can load onto the users' computers. Alternatively, the analytics features can be provided as a web-based application.

In an example embodiment, the system is configured to allow a user to limit access to the data associated with the user, e.g., data created by the user or about the user, to only certain users (e.g., only one or more certain specified users or only users of one or more certain specified user groups) or to only certain systems (e.g., only via the Guide system). For example, the system can require entry of a user ID by which the system determines whether a user is authorized to access such restricted data.

It is noted that the analytics software and the input and/or output data can be at different locations.

## Analyses of the SFM Analytics:

### 1. Population overlap:

According to an example embodiment of the present invention, the SFM analytics system obtains as input a group of VOAs from a population of patients, where each VOA is associated with a measure of effectiveness, and the SFM analytics system determines a target volume based on the input information. For example, a collection of VOAs can form a group to be subjected to the analytics calculations where there is a clinically significant commonality between the VOAs of the collection. For example, VOAs of patients who share a certain diagnosis, e.g., the patients all have Alzheimer's disease, can form a clinically significant group for analysis to determine a target volume for treating Alzheimer's disease. Another or alternative significant commonality can be that the VOAs are associated with similar anatomical locations at which the stimulation electrode is implanted.

According to an example embodiment, the system is configured for a user, e.g., a physician, to manually create a group of VOAs to be used as input to analytics functions. For example, a physician might notice that a number of VOAs of stimulation settings applied to one or more patients provided excellent results, and the user can form a group for such VOAs. A single VOA can be a part of multiple groups.

In an example embodiment, for the analysis of a group of VOAs, the system places all of the VOAs in a common spatial reference space. For example, the system is configured to transform one or more, e.g., all, of the VOAs into a common atlas space. This may be required for example where the VOAs were obtained for different patients whose anatomical makeup varies.

The system is configured to determine where the VOAs of the common reference space spatially overlap for determining a target volume. For example, the system can output a combination of a collection of points, e.g., voxels, that belong to all or a threshold number or percentage of the VOAs of the group as a target volume. In this regard, VOAs each encompass a collection of points, e.g., voxels. According to an example embodiment, the system provides a user interface configured for user interaction therewith to input the threshold number or percentage.

Alternatively, the system can perform more complex calculations for finding voxels of significance shared by some of the group of VOAs, the combination of voxels of interest forming a target volume.

For example, in an example embodiment, the system is configured for obtaining ratings for each of the group of VOAs, e.g., one or more ratings for some characteristic, e.g., therapeutic effect, side effect, or how well it helped the patient with respect to some score. For example, clinicians can input into the system a rating on how well the patient is doing against some standardized scale.

The system is configured to assign a greater weight to those VOAs associated with better scores, e.g., the greater the score, the greater the weight. For example, the system can weight each point, e.g., voxel, within a VOA by the VOA's score. Then the system calculates for each of the points within any one of the group of VOAs, a respective score based on the combined weighted scores of the point across all VOAs of the group that include the respective point. For example, the system can sum the scores for each of the points. Alternatively, the system can average the scores for each of the points.

According to the latter embodiment, the system also takes into consideration the number of VOAs in which the point is included. For example, a score of 0, a negative score, or some other value can be assigned to a point for a VOA in which it does not appear. Alternatively, the number of VOAs of the group in which the point is included can be considered as a separate factor in the calculation. According to an example embodiment of the present invention, the system compares each point's value to a threshold, and includes a point as part of an output target volume if the point's score meets the threshold. According to an example embodiment, the system provides a user interface configured for user interaction therewith to input the threshold score. According to an example embodiment of the present invention, the system selects a threshold number or percentage of highest scoring points as the target volume. According to an example embodiment, the system provides a user interface configured for user interaction therewith to input the threshold number or percentage.

According to an alternative example embodiment of the present invention, the above described thresholding can be performed to produce a volume which the system can subject to further calculations from which to select a target volume for output.

## 2. Group Comparisons:

In an example embodiment of the present invention, the system is configured to compare different groups of VOAs to determine target volumes of stimulation and/or volumes to be avoided, e.g., so as not to produce an unwanted side-effect. VOAs can be associated with respective therapeutic effects and/or side effects, including by degree of severity. For example, such associations can be based on user input provided using user interfaces such as those described for example n U.S. Pat. App. Ser. No. 14/212,730, filed March 14, 2014 and U.S. Prov. Pat. App. Ser. Nos. 61/793,773 filed March 15, 2013 and 61/830,855 filed June 4, 2013. Effects, associated with the VOAs, can also be obtained based on sensor information obtained during stimulation or in a period immediately following the stimulation. In an example embodiment, the associated effects are used for obtaining relevant volumes on which basis to generate new target volumes and/or side effect volumes.

For example, a first collection of VOAs associated with a certain side effect can form a first VOA group and a second collection of VOAs not associated with the side effect can form a second group. The system can automatically create these groups. Alternatively, a user can manually form the groups and input an instruction to the processor to perform a comparison. Similarly, the system and/or the user can select certain therapeutic effects as the characteristic by which to group VOAs. For example, an improvement in motor skill or a motor skill score can be set as a therapeutic effect by which to group VOAs.

The system is configured to transform the VOAs to a single common atlas space and find an area included in the group associated with the side effect (or therapeutic effect) and not included in the group that is not associated with the side effect (or therapeutic effect), and output the area, e.g., in relation to an atlas space, as an area that should not be stimulated. According to an example embodiment, more than one side effect and/or more than one therapeutic effect can be combined for the groupings, e.g., as a logical OR (e.g., any VOA associated with any one or more of certain specified side effects or therapeutic effects) or as a logical AND (e.g., any VOA associated with the combination of specified side effects or therapeutic effects). According to an example embodiment, the system is configured for use of a combination of side effect and therapeutic effect information for grouping VOAs in analytics for selecting a target VOA, by combining therapeutic effect areas and subtracting therefrom the side effect areas. According to an example embodiment of the present invention, the system provides a user interface configured for user interaction therewith to input a threshold degree of the side effect and/or therapeutic effect required for consideration of the respective VOA as part of the group.

Alternatively, a more complex calculation can be used to determine the points of the area to be avoided. For example, each point can be individually scored based on a combination of scores of all VOAs of the group associated with the side effect and in which the respective point is included, e.g., where the score for a point within a VOA depends on the severity of the side effect for that VOA. In an example variant of this embodiment, the system first finds which areas are included in a threshold number and/or threshold percentage (e.g., 100%) of the VOAs associated with the side effect and not included in a threshold number and/or percentage (e.g., 100%) of the VOAs not associated with the side effect. For voxels in the identified area, the system assigns respective scores based on the severity of the side effect for the VOAs in which the voxel is included. Alternatively, for each voxel, the system assigns a score based on a combination of those VOAs associated with the side effect an in which the voxel is included and of those VOAs not associated with the side effect. For example, inclusion in a VOA associated with the side effect can contribute to a higher side effect score, the extent by which the score is raised being dependent on the quantified severity of the side effect with which the VOA is associated; and inclusion in a VOA not associated with the side effect can contribute to a lowering of the score of the voxel. The system includes those points whose combined score meets a predetermined threshold, or a threshold number or percentage of points sorted by score, as the volume to be avoided.

Similarly, in an example embodiment of the present invention, where a benefit is associated with a first group of VOAs and is not associated with a second group of VOAs, the system finds the points, e.g., voxels, that are included in the VOAs of the group associated with the benefit and not included in the group that is not associated with the benefit, or scores points by the extent of their inclusion in one group over the other group, as described above, to output the combination of such points as a target volume.

According to an example embodiment, the system can, based on the VOAs of the patient population and their corresponding clinical effects, determine on a voxel-by-voxel basis, which voxels contribute to a certain effect, and combine those voxels into a target or side-effect volume. For example, if a voxel is included equally in both VOAs associated with a particular clinical effect and VOAs not associated with that clinical effect, then the voxel can be considered not to statistically significantly contribute to the particular effect. On the other hand, if a voxel is included statistically significantly more in VOAs associated with the effect than those not associated with the effect, then the voxel can be treated as being one that helps contribute to the specified effect when stimulated.

According to an example embodiment, the patient population VOAs and their effects can be used to construct electric field maps on a voxel-by-voxel basis with corresponding scores, for example, as described in U.S. Pat. App. Ser. No. 13/600,855 filed August 31, 2012, to obtain a value for each voxel with respect to the specified effect.

According to an example embodiment, the system is configured to generate a probability voxel map that maps the probability that stimulation of each respective voxel will contribute to the specified effect, the system then selects all voxels whose probabilities meet a predetermined or user-input threshold probability, and the system then draws a three-dimensional surface outline around all the voxels meeting the threshold which are at the extremities to form the target or side effect volume. In an example embodiment, with further information concerning the leadwire location, the system is configured to output the stimulation parameters for stimulating the thus generated target region or for avoiding the thus generated side effect region.

In an example embodiment, a physician inputs desired therapeutic effects vs. side effects. Those therapeutic effects and side effects are weighted and overlaid / summed. A 3D voxel map is generated. A lead trajectory is placed in this space. Possible VOAs based on that lead trajectory are predicted and scored. The best score, weighted against needed settings, is chosen, and final settings proposed. For example, for each VOA/contour, map to a field, then inverse from field to programmed amplitude / pulse width settings. To choose a VOA, use sphere on radius r, placed along lead at spacing dx, and choose location x for sphere with maximal score.

3. VOA Steering Based on Clinical Effects / Indications / SFM Analysis:

According to an example embodiment, the system provides a user interface for input of desired therapeutic effects and/or undesired adverse side effects to be considered, e.g., in the form of a questionnaire. Once the questionnaire is completed and submitted, e.g., by the press of a "submit" button, the system outputs a suggested set of stimulation parameters corresponding to a VOA and/or output the suggested VOA. For example, the system analyzes prior VOAs, e.g., as explained above, to determine the regions associated by the correct VOA corresponding to the indicated effects. According to an example embodiment, the system also provides for alternatively receiving input of indications of a patient, and the system finds the VOAs of the patient population corresponding to stimulations performed for other patients with the indicated indication(s), and outputs the overlapping (or other combination) VOA regions. According to an example embodiment, the system is configured to receive as input a set of indication(s) and clinical effect(s), and use the combination to filter the VOAs for determining and outputting a target volume.

According to an example embodiment, the system includes an input device such as a dial, as a hardware component or as a soft input device displayed as a user-interactive graphical component in a user interface, by which the user can input a change in a desired clinical effect or input a degree to which to consider an adverse effect to be avoided. For example, the user can turn a dial that is associated with tremor to indicate the degree to which the recommendation of a target stimulation location should be based on obtaining the desired therapeutic effect of reducing tremor.

In response to user interaction with the input device, e.g., in response to the user turning the dial, the system outputs the next closest program to result in that input change. The change in program for the desired change in clinical effect can be with respect to location of stimulation, pulse width, frequency, amplitude, etc.

An adjustment in some parameters to obtain a greater degree of the indicated therapeutic effect can result in an increase of adverse effects. Thus, in an example embodiment, the system determines the nearest VOA to result in the increased indicated therapeutic effect without the increase in the adverse side effect, or, where no such VOA exists, the one with the least increase in adverse side effect. Accordingly, in an example embodiment, the system finds the VOA closest to the VOA associated with the current settings that is also associated with the desired effect and not associated with the adverse effect. The system then outputs the program settings associated with the VOA closest to the VOA associated with the presently indicated stimulation settings, or, according to an alternative example embodiment, the VOA associated with the settings most similar to the presently indicated settings, where the modified settings produce the indicated desired change in clinical effect.

According to an alternative embodiment, the system determines the VOA for producing the greatest amount of the indicated desired change, determines a trajectory of possible VOAs between the one corresponding to the presently indicated settings, and, as the user turns the dial, the system outputs those VOAs and/or corresponding stimulation parameters, in succession until the optimal VOA is reached. According to an example embodiment, the system is configured to output a visual of the VOA path from the current location to the optimal location. For example, the system simultaneously outputs the current and optimal VOAs, as well as those in between, each for example being demarcated by a respective outline. In an example embodiment, as the user turns the dial, a different one of the displayed VOAs in the path is highlighted, and its associated parameters displayed.

Thus, for example, a presently set stimulation program is defined (partially) by amplitude, pulse width, rate, and location of stimulation pulses. A control, such as a dial, is provided for a corresponding effect or symptom. For example, a dial can be provided for tremor control. As the user turns the dial, the system auto-adjusts the parameters, e.g., auto-adjusts the amplitude setting, to gradually evolve the VOA corresponding to the current settings to the VOA associated with optimal tremor control, with each incremental turn of the dial moving the VOA another step along a tremor improvement path that extends from the current VOA location to that corresponding to the optimal tremor control.

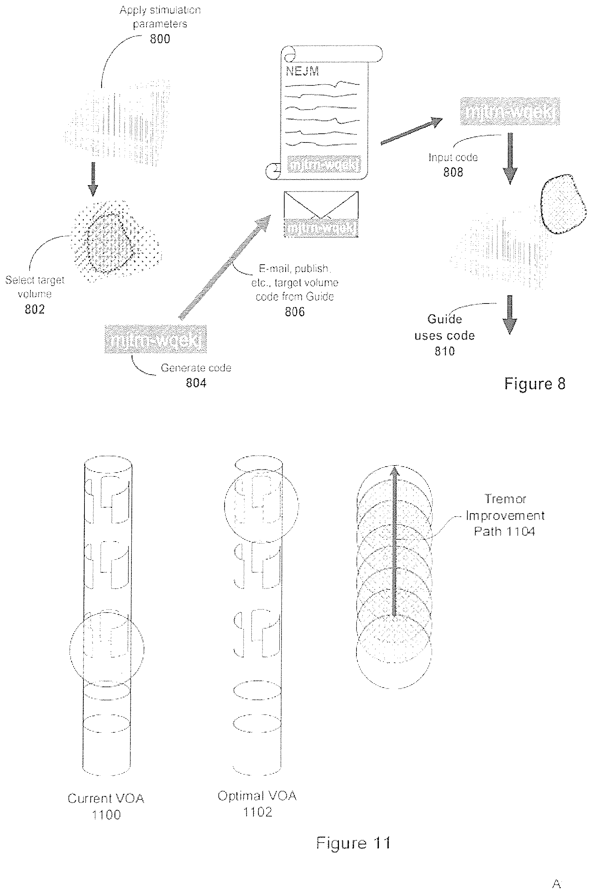

Figure 11 shows an example current VOA 1100, an Optimal VOA 1102 for improvement with respect to tremor, and a tremor improvement path 1104 outlining a trajectory from the current VOA 1100 to the Optimal VOA 1102.

Intermediate locations along the path can be associated with adverse side effects. According to an example embodiment, the system is configured to output warnings when intermediate VOAs are associated with causing adverse side effects. In an example embodiment, if multiple trajectories can lead from the current VOA to the optimal VOA, for tremor control for example, the system is configured to select the trajectory that avoids VOAs associated with adverse side effects, or that entails fewest VOAs associated with adverse side effects (even though the trajectory is not the shortest of the trajectories). According to an alternative example embodiment, the system is configured to skip over VOAs associated with any adverse side effects, or alternatively VOAs associated with severe side effects.

According to an example embodiment, the system is configured to alternatively or additionally perform the reverse, where the user inputs a stimulation program, and the system determines, based on the VOAs of the patient population and their corresponding indicated clinical effects, the expected therapeutic and/or adverse side effects of the indicated program. The system then outputs the expected clinical effects.

According to an example embodiment, in addition to outputting the expected clinical effect, the system is also configured to determine and output expected effects with certain slight modifications to the stimulation parameters. For example, in an example embodiment, the system outputs a message stating that if the stimulation is shifted a bit higher or rotationally around the leadwire by a certain number of degrees in a particular rotational direction, etc., then a particular specified change in clinical effect would be obtained. In an example embodiment, the system limits such output to minor changes, e.g., movement of the stimulation up to a certain distance upward or downward, or a certain number of degrees around the leadwire, or a certain threshold additional or lesser distance radially outward from the leadwire, etc. Alternatively, the system outputs the next expected change in effect for each of one or more of such changes regardless of the extent of the change, e.g., the next change in effect expected with a shift of stimulation upward regardless of the amount of such upward shift. The system or user may define the size of the minor change and may be, for example, a change of 1, 2, 5, 10 percent or more in the stimulation parameter.

According to an example embodiment, a user can use a user interface of the system to steer stimulation, for example as described in U.S. Pat. App. Ser. No. 14/011,836 filed August 28, 2013 and/or in U.S. Prov. Pat. App. Ser. Nos. 61/693, 866 filed August 28, 2012, 61/699,135 filed September 10, 2012, 61/699,115 filed September 10, 2012, and 61/753,232 filed January 16, 2013. In an example embodiment, as the user shifts the stimulation, the system checks the patient population VOAs to determine whether the steered change shifts the VOA estimated for the new steered settings towards a location associated with an adverse effect, in which case the system outputs a warning signal, e.g., visually, haptically, and/or aurally. For example, according to an example embodiment, a signal is gradually increased as the VOA is steered closer to, or to cover more of, the area to be avoided.

4. Group Analysis Considering Biological Networks:

The body includes various biological circuitries that biologically relate various parts of the body to each other in various respective ways. For example, as described in U.S. Prov. Pat. App. Ser. No. 61/904,248 filed November 14, 2013, stimulation of one anatomical region can cause an effect in another anatomical region. Thus, according to an example embodiment, where there are VOAs grouped, for example by similar therapeutic or side effect regions, the system is configured to, for one or more particular biological circuitries, determine an overlap (if any) by a significant number of the VOAs with a shared component of the considered biological circuitry for generation of a target or side effect biological component with respect to stimulation. For example, in an example embodiment, the system is configured to determine whether a threshold number or percentage of VOAs that are associated with a particular therapeutic effect or side effect overlap a shared neurological fiber (even if the VOAs are in non-overlapping anatomical regions), and if so output an indication of that biological fiber as a target fiber or a fiber to be avoided (depending on whether it is associated with a therapeutic effect or adverse side effect). In fact, all of the features described herein with respect to determination and output of target or side effect volumes can also be used for determining and outputting target or side effect biological structures such as neurological fibers (for example, instead of returning a target volume in response to the input indications and/or clinical effects to be considered, the system can return a target of one or more fibers).

According to an example embodiment, the system uses a combination of region overlap and biological circuitry overlap for generation of a target volume or side effect volume. For example, the system generates the volume based on anatomical region overlap of the volumes, but only those volumes which overlie a shared biological component of a considered biological circuitry are grouped into a new target or side effect volume. For example, if neurological fibers are considered, then the VOAs associated with a same, for example, therapeutic effect are combined to form a new target volume conditional upon that they both overlie the same neurological fiber (of the respective patients with which they are associated).

According to an example embodiment, the system displays putative functional subdivisions of a relevant anatomical region, e.g., the STN, and the putative connections between the anatomical region, e.g., the STN, and other regions, e.g., the pallidum, pallidum and thalamus, thalamus and cortex etc. When a VOA is activating a particular part of the STN, the system lights up corresponding nodes along the circuit and pathways within the biological circuit. In an example embodiment of the present invention, the system provides the display in a user interface in which a user can click the pathways, in response to which the system displays links to literature discussing the relevant biological connections. In an example embodiment, the system provides this information in a wiki in which the plurality of users of the system can provide additional source and explanatory information concerning the biological connections.

In an example embodiment of biological network/circuit analytics, fibers can be stimulated at different locations along some fiber(s). System knows that two points or structures are in some biological circuit or different points along the same fiber tract, and thus related and may have the same primary effect (perhaps with different secondary effects). For example, when planning an implantation and stimulation target, if the clinician desires to stimulate target A, but it is not possible (for example, other structures are in the way of target A), a target C that produces the same primary effect can be used and, therefore, implant in target C.

As another example, after implantation, the clinician desires to stimulate target A, but cannot because the lead is not in the necessary location, a target C that produces the same primary effect can be used and, therefore, program stimulation towards C. In these examples, the analytics determine that targets A and C have been shown to produce the same (or similar) effect, although they are spatially non-overlapping. Target volumes often reduce to a point but with, for example, DTI data, target volumes can reduce to a line.

5. Group Analysis Considering Biological Signals:

There are many types of brain signals corresponding to respective brain states, including for example, electrophysiological brain states. Examples of such signals include MER and Local Field Potential (LFP). Such signals can be location specific, e.g., to particular parts of the brain. Similarly, components in medical images can correspond to respective brain states, and therefore medical images, such as, for example, fMRI, PET and SPECT, also correspond to respective brain states. Such brain state data can be obtained and associated with patients and their respective VOAs. For example, the brain can be stimulated while also obtaining signals, such as MER, PET, EEG, and/or neuro-activation spikes. According to an example embodiment the system is configured to use such signals as a signature with which the VOAs are associated, for the purpose of VOA groupings, similar to that which is described above with respect to using the same therapeutic effect(s) and/or side effect(s). A single brain state can be represented by a combination of signal types. Where a number of VOAs are associated with a selected signal signature, the system is configured to generate a volume by the intersection (or union) of those volumes. For example, the system can generate a plurality of volumes each associated with a different respective MER signal signature. If there is a desired brain state for a patient, the user can enter the relevant brain state, e.g., MER signature, in response to which the system outputs a volume that is based on the VOAs associated with the indicated signal signature. Indeed, over time, doctors/clinicians can come to associate certain biomarkers with desired therapeutic effect, and therefore use such markers for the analysis of the VOAs of the patient population.

In an example embodiment, location-specific data on brain response to respective VOAs, for example as indicated by changes to location-specific biomarkers in the brain, is aggregated over a large number of patients. For treatment of a new patient, the system is configured to obtain biomarkers characterizing a current brain state of the patient. The system is configured to obtain biomarkers representing a desired brain state for the patient. The system is then configured to automatically determine and output a target volume of activation estimated as most likely to achieve the change in brain state from the current brain state to the desired brain state. For this determination, the system is configured to search the library of VOAs and the corresponding aggregated location-specific brain response data to determine which VOA has been shown to produce the response which provide the desired change.

For example, in an example embodiment, information of a new patient, including a corresponding, e.g., novel, indication is entered. A biomarker is found. Brain states are searched. The system then identifies the brain state which would abolish the biomarker. The system then identifies the VOA associated with that brain state, e.g., the VOA which would produce the desired change from the current brain state to produce the identified target brain state. The system then provides that VOA (and associated stimulation settings) as a target volume for treating the entered indication.

## 6. Filtering VOAs based on Medications or Time of Day:

The medication a patient is on and the time of day when stimulation is performed can affect stimulation results. According to an example embodiment, the system filters the VOA groupings by the medications which the patient records indicate the patients, to whom the respective VOAs correspond, to have been on at the time of the stimulation. For example, a user can input a medication which a current patient is on, and request a target stimulation volume based on region/voxel combinations of VOAs corresponding to patients on the same medication (and/or dosage). In an example embodiment, the system is configured to consider medication concentration, type, and/or state.

According to an example embodiment, the system filters the VOA groupings by the time of day when the stimulations corresponding to the VOAs were performed. For example, a user can input a time of day (e.g., morning, afternoon, early evening, night), and request a target stimulation volume based on region/voxel combinations of VOAs corresponding to stimulations performed during the relevant time period.

Thus, example embodiments provide for use of a plurality of parameters as filters for analytics. Time or medication concentration/type/state are usable to refine analyses. For example, unfiltered data may show a weak correlation between a particular stimulation volume and an effect, but, upon filtering by time, the data may show a strong correlation between the stimulation volume and the effect when the stimulation is performed in the morning. In an example embodiment, the system is configured to use the filtered analyses to inform future program settings to compensate for changes in time. Similarly, other potential parameters for filtered analyses include, for example, demographics, health state, body position, and/or disease type or subtype.

## 7. SFM Analysis for Target Volume Generation Based on Selected Clinical Effects:

VOAs can be generated for a plurality of patients of a patient population, and each of all or a subset of the VOAs can be recorded in association with one or more therapeutic effects and/or adverse side effects. In an example embodiment, the system provides a user interface via which a user, e.g., a clinician, can select one or more therapeutic effects, in response to which the system outputs a target volume for achieving the indicated combination of effects based on analysis of the VOAs of the patient population and the therapeutic effects with which they are associated, where only those VOAs which are associated with the indicated therapeutic effects are selected. The system then outputs a volume formed by the overlap of those VOAs or formed of the voxels that are in a significant number of the VOAs associated with that therapeutic effect. In an example embodiment, the system provides a user interface via which the system is configured to receive user input of a threshold number or threshold percentage of considered VOAs in which a voxel must be found to be included in the returned area.

Similarly, according to an example embodiment, the system provides a customized side effect volume showing regions to avoid, based on user selected adverse side effects, where the system returns a side effect volume formed by the overlapping of VOAs associated with the indicated side effects, e.g., subtracting therefrom regions in VOAs not associated with the adverse side effects. According to an example embodiment, the user can input both therapeutic effects and adverse side effects to consider, and the system produces a new target volume formed by the overlap of the VOAs associated with the indicated therapeutic effect minus portions associated with the volumes associated with the indicated adverse side effects. Similar to that which is described above, in an example embodiment, the system provides a user interface via which the system is configured to receive user input of a threshold number or threshold percentage of considered side effect VOAs in which a voxel must be found to be considered as belonging to the side effect area.

For example, a user can input a desired primary effect, in response to which the system suggests one or more stimulation target volumes and/or associated stimulation parameters. The user can continually modify and refine the desired effects. For example, the user can input a request for more of a particular clinical effect and/or avoidance of additional side effects, in response to which the system outputs a modified target, until finally the system outputs a suggested final target volume or family of volumes of a circuit.

In an example embodiment, various target effects/structures can be used to guide a trajectory through multiple targets. Select a series of desired effects, and the system, in an example embodiment, shows one or more trajectories through multiple targets that may have disparate results related to the desired effects.

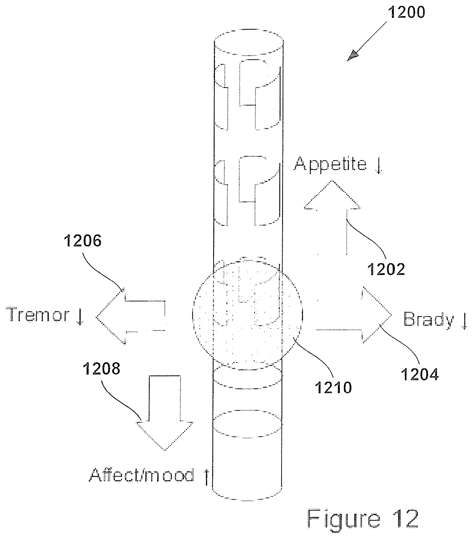

In an example embodiment of the present invention, a user can input stimulation program settings in response to which the system outputs a display in the graphical user interface (GUI) a map of neighborhood effects showing the respective next change in effects associated with a change in the VOA position, in each of a plurality of directions, from the current VOA position. For example, For example, Figure 12 shows an example of a neighborhood effects map 1200 with labels 1202, 1204, 1206, 1208 indicating the change in an effect with a change in the VOA 1210 position. In an example embodiment, users can build the neighborhood effects probability atlases, and the users can export, share, and import them.

8. Clinical Effects Analysis for Target Stimulation Time and/or Medication Determination:

According to an example embodiment, the system analyzes the effects of stimulations against time of day to determine a target stimulation time. For example, the system creates a histogram of stimulations by time of day (e.g., morning, afternoon, early evening, night) to determine if a particular therapeutic effect or side effect is statistically significantly associated with a particular time of day, and, if so, outputs such information. This information can differ for different considered therapeutic effect and/or side effects. According to an example embodiment, the system provides an interface in which the user can select combinations of therapeutic effects and/or side effects, and the system determines whether there is a statistically significant time period association for that indicated combination, in which case, the system outputs a recommended time of day for therapy.

Medications taken by respective patients with which the stimulations are associated can be considered in a similar manner to determine a recommending medicinal regimen to follow during the period when stimulation is performed.

The affect that time of day and/or medication has on the clinical effects may be dependent on the particular areas stimulated and/or the demographics of the patients subject to the respective stimulations. Thus, according to an example embodiment, the system tests the statistical significance of time of day and/or medication against different stimulation areas and/or demographics. Thus, different times of day and/or medications can be recommended for different stimulation regions and/or for different patient demographic information.

9. Analysis of VOA Overlap with Target Volume:

According to an example embodiment of the present invention, the system is configured to quantify the extent to which a VOA meets a target volume based on a spatial difference of the VOA and the target volume. For example, the extent to which the VOA extends beyond the target volume, the extent to which the target volume extends beyond the VOA, and the extent to which the VOA and the target volume overlap contribute to an overall score of the VOA. See, for example, U.S. Provisional Patent Application Serial Nos. 61/521,572, filed August 9, 2011 and 61/549,053, filed October 19, 2011.

According to an example embodiment of the present invention, the system is configured to determine how well a group of VOAs meets a target volume. For example, the system determines a spatial difference between the group of VOAs and the target volume. For example, the system assigns to each VOA of the group a score indicating how well the respective VOA meets the target, and a combination of the scores can be used as a metric to determine how well the group meets the target volume. For example, a particular set of parameter settings might be common to all of the VOAs of the group, and it may be useful to know the extent to which the settings are expected to produce a VOA that substantially meets the target volume. The score can be used as a metric of the expectation.

Alternatively or additionally, the determination of the extent to which the group of VOAs corresponds to the target volume can be performed for the VOAs as a whole, by which the system calculates a mean and/or standard deviation to rate the VOAs as a group.

According to an example embodiment, the system outputs a number indicating how many or the percentage of the VOAs of the group that meet a certain threshold correspondence with the target volume.

According to an alternative example embodiment, for each point that is included in any of the VOAs of the group, the system determines the number of the VOAs of the group in which the point is included and includes the point in a composite volume if the point is determined to be included in a threshold number or percentage of the VOAs of the group. The system compares the composite volume to the target volume and scores the composite volume based on the degree of similarity between the target and composite volumes. The system outputs the score as a rating of the correspondence between the group of VOAs and the target volume.

According to an example embodiment of the present invention, a similarity of an average center of mass of the group of VOAs and the center of mass of the target volume is a factor used by the system to calculate the rating of the correspondence of the group of VOAs to the target volume.

10. SFM Analysis for Patient-Specific Atlas Generation:

According to an example embodiment of the present invention, the system analyzes VOAs of a plurality of patients to create a new patient population group for obtaining an average atlas to be registered to a new patient, e.g., as described in the '232 application. Clinicians program a leadwire implanted in a patient to obtain certain therapeutic effects, while avoiding adverse side effects as much as possible. Different patients will require different volumes of tissue to be activated for achieving results tailored to that respective patient, because different patients need different therapies and because, even for a same therapy, different patients can require different stimulations for achieving the same desired results. If, despite the tailoring of parameters and VOAs to achieve customized patient-specific results, the VOAs of a group of patients are similar, in an example embodiment, the system forms a new patient population group, which is the group of patients with whom the similar VOAs are associated. For example, in an example embodiment of the present invention, the system compares the VOAs to each other to find significant overlap and/or lack of significant spill. For example, in an example embodiment, for comparison of the VOAs of the patient population to each other, the system uses one or more equations described, for comparing a VOA to a target volume, in U.S. Pat. App. Ser. No. 13/570,736 filed August 9, 2012, which claims priority to U.S. Prov. App. Ser. Nos. 61/651,282 filed May 24, 2012, 61/549,053 filed October 19, 2011, and 61/521,572 filed August 9, 2011. Alternatively, the system can use an Overlap Ratio Measure, for example as described in Siegel et al., "Spatiotemporal Dynamics of the Functional Architecture for Gain Fields in Inferior Parietal Lobule of Behaving Monkey," Cerebral Cortex 17(2), pp. 378-390 (February 2007).

In an example embodiment, if the system finds a group of patients within the patient population whose successful VOAs are similar, for example based on the mentioned comparisons, the system generates a new patient population group the members of which are the patients whose corresponding VOAs have been determined to be similar.

In an example embodiment, the new patient population group is used for obtaining a customized atlas for registration to a new patient, e.g., as described in the '232 application. For example, the system determines recorded characteristics, e.g., indication, age, sex, geographic location, medications being taken, etc., of the patients of the new patient population group which are shared by all or a threshold percentage of the new patient population group. If characteristics of a new patient match those characteristics (or a significant number of those characteristics), the system uses an atlas from that new patient population group to register to the new patient, e.g., to a medical image of the new patient. Alternatively, a doctor, surgeon, and/or clinician can view the characteristics associated with various patient population groups and, for example, based on such information, manually select the patient population group to use for obtaining an atlas to register to the new patient. The atlas of the patient population group used for registration to the new patient can be, for example, an average of the atlases of the patient population group, and average medical image of the patient population group (e.g., normalized to a common reference coordinate system), or an atlas generated based on the average image.

According to an example embodiment, the group can be further divided by noted associated therapeutic effect. For example, if of 100 similar VOAs, 50 are associated with a first therapeutic effect and 50 are associated with a second therapeutic effect, then according to an example embodiment, the system generates a first patient population group formed of the patients associated with the first 50 VOAs and generated a second patient population group formed of the patients associated with the second set of 50 VOAs. For atlas registration, the system (or clinician/doctor/surgeon) can select the set associated with the therapeutic effect which is desired to be achieved in the new patient.

According to an example embodiment, the system may similarly form new patient population groups based on similarities in recorded adverse side effect volumes. For example, if VOAs of a plurality of patients which were associated with side effects are recorded, the system is configured to, in an example embodiment, group those patients into a new group, which are then selected for use to create a patient-specific atlas for a new patient, based on similarities between the characteristics of the new patient and that patient population group. As described above, these groupings can be further divided according to type of side effect.

According to an example embodiment of the present invention, the system uses a lack of similarity of VOAs as a factor to determine whether to create a new patient population group. For example, if a group of patients have certain commonalities, e.g., one or more of age, race, geographical location, indication, medications takes, etc., which commonalities would suggest that similar VOAs should be used for those patients, but instead the VOAs for those patients actually break down into two or more groups, where the VOAs in the same one of the groups are similar, but are significantly different than the VOAs in the other groups, then the system forms a new patient population group for each of those groups of VOAs. When an atlas is to be generated for a new patient, in an example embodiment, the system compares an image of the new patient to, for example, average atlases or images of each of the groups, and selects the average atlas or image of the group most similar to the image of the new patient, for registration to the patient for generation of the patient-specific atlas.

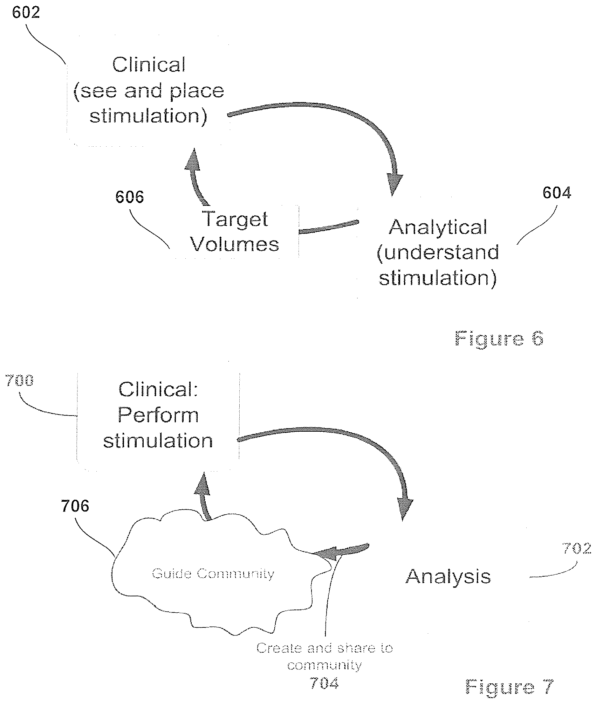

Thus, as shown in Figure 6, during a clinical stage 602, a user, e.g., a doctor, patient, clinician, etc. can use a stimulation programming module (or modules) to set stimulation settings to stimulate an anatomical region of a patient. Many users can do this for many patients, e.g., at a same or at a plurality of terminals. The module is configured to generate estimated VOAs corresponding to the stimulation settings. The module is further configured to receive user and/or sensor input regarding clinical effects of the stimulation, which the system stores in association with the respective VOA on a cloud-based data store. The system is further configured to obtain additional patient information, e.g., from an electronic medical file, with which information the VOA is associated. An analysis module 604 performs analyses on the plurality of VOAs, the associated clinical effects, and/or the associated patient information, to refine target and side effect volumes. The user of the programming module can then access the refined target and side effect volumes 606 for tailoring stimulation settings of subsequent stimulations for a plurality of patients. The target and side effect volumes used for the subsequent stimulations can be based on a filtered set of the prior VOAs, e.g., which set is most relevant to the respective patient to whom the subsequent stimulation is being applied. However, aside from use of the VOA analytics to refine target and side effect volumes, in an example embodiment, the system is configured to identify clusters of patients of the patient population based on the respective VOAs associated with respective ones of the patients of the patient population, and identified relationships between such VOAs, where the clusters form respective patient population groups for which respective atlases are provided.

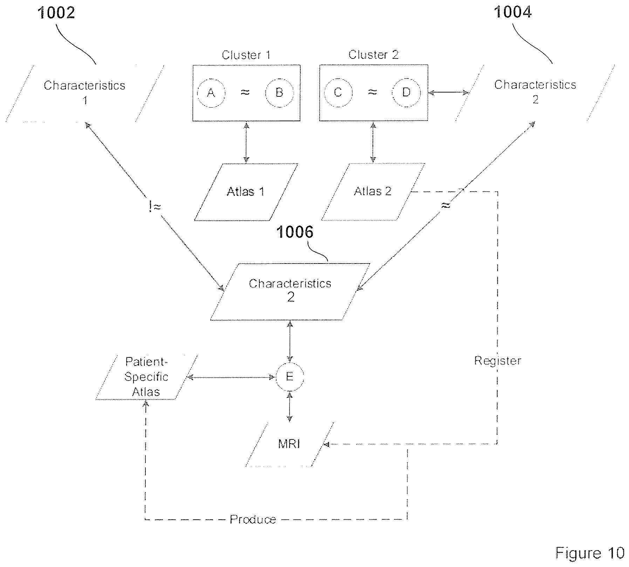

For example, Figure 10 illustrates an example where the data store includes VOAs corresponding to stimulation programs set for patients A-D, MRIs of the brains of patients A-D, and other medical record information concerning patients A-D. In the illustrated example, the system identified an overlap between the VOAs associated with patients A and B, and identifies an overlap between the VOAs associated with patients C and D. The system therefore generates Cluster 1 formed of patients A and B, and combines data of the MRIs of patients A and B to form Atlas 1 for Cluster 1; and generates Cluster 2 formed of patients C and D, and combines data of the MRIs of patients C and D to form Atlas 2 for Cluster 2. The system also identifies characteristics, e.g., medical record characteristics, shared by patients A and B and characteristics shared by patients C and D, and associates the respective shared characteristics with the respective clusters.

The system is configured to provide user interfaces for stimulation programming and visualization in which the system outputs a graphical representation of an implanted leadwire and/or stimulation volumes, such as estimated VOAs, target volumes, and/or side effect volumes, relative to anatomical structures of the patient, where those anatomical structures are spatially arranged according to a patient-specific anatomical atlas. Therefore, when a new stimulation programming/visualization electronic record for a patient is generated including the patient information used for outputting patient-specific stimulation volume information, the system is configured to store in the electronic record the patient-specific atlas. To do so, in the example illustrated in Figure 10, the system is configured to select one a plurality of patient population atlases for registration to the new patient. In Figure 10, the system compares characteristics, e.g., medical record characteristics, which can include, for example, demographics, of new patient E to the respective characteristic sets associated with each of Clusters 1 and 2. In the example shown in Figure 10, the system has determined that the characteristics of new patient E are most similar to those associated with Cluster 2, and the system therefore selects Atlas 2 of Cluster 2 and registers it to anatomical information, e.g., an MRI of new patient E to generate a patient-specific atlas for patient E.

In an example embodiment, referring to the example illustrated in Figure 10, subsequent to applying stimulation programs to patient E, patient E can be added to the patient population, and the system is configured to further modify the previously generated clusters in view of VOAs associated with patient E, for example to add patient E to one of the previously generated clusters, to add a new cluster, and/or to break apart one or more previously generated clusters. Thus, the patient population clusters can evolve over time. With each change to the patient population clustering, the system can generate a new respective patient population atlas for each new and/or modified cluster, which the system is configured to use for later generation of patient-specific atlases for newly added patient records. In an example embodiment, the system is configured to update even the patient-specific atlases previously generated for patients, e.g., even ones previously included in a cluster, based on a modification to the patient population clusterings.

## 11. Patient-Specific Atlas Generation Based on a Hierarchy of Images or Atlases of a Patient Population:

According to an example embodiment of the present invention, take the MRI of many patients and either using the whole MRI or regions of interest within it (e.g., Basal Ganglia), cluster the images (or corresponding atlases) using some metric of image similarity, e.g., to create a hierarchy of medical images or atlases. For example, the clustering of the database images may be done hierarchically so as to obtain a cluster tree, where, at the bottom of the tree, the individual images are the leaf nodes, which can then branch upward to a node of a higher hierarchical level. The nodes of the higher hierarchical levels correspond to a template constructed based on the images/templates of nodes of the branches below the respective node.

In an example embodiment, the hierarchical tree is used for efficiently finding an image/atlas to register to a new patient. For example, the system compares an image of a new patient to the average image/atlas of the nodes beginning with the top hierarchical level, working downwards until branch by branch, at each level selecting the node with the greatest match, until selecting the best leaf node. In this manner, the new patient's image need not be compared to each leaf node to find the best match. The selected leaf node's atlas can then be registered to the image of the new patient to form the patient-specific atlas. This may enable an efficient search for similarity between the patient and the candidates.

In an example embodiment, the system selects from the hierarchy the 'n' closest MRIs transformed to a common space, e.g., Montreal Neurological Institute (MNI) space and/or takes the average MRI (or other mathematically constructed MRI combination) that best represents the 'n' MRIs, and the system registers the selected MRI/Atlas for registration to the new patient's medical image.

## 12. Interface for User Input of Logical Combinations of SFMs for Target and/or Side Effect Volume Generation:

According to an example embodiment, the system provides a user interface by which the user can select characteristics to consider when generating a target and/or adverse side effect volume, and to also select a logical operator to use for the generation of the new volume. For example, the user can input an instruction to a return a new volume based on all therapeutic VOAs associated with one or more therapeutic effects and/or one or more identified patient characteristics with which the VOAs are associated, and further select an operator such as union or intersect. For example, the user can select a union of VOAs of a certain type, in response to which the system returns a volume composed of the combination of areas in the relevant VOAs, or the user can select an intersection of volumes, in response to which the system returns a volume composed of the areas found in all (or a threshold percentage) of the relevant VOAs. Another logical operation can be NOT, where the user can indicate certain VOAs not to be considered (for example not VOAs associated with particular specified patients, not patients of particular specified doctors, and/or not patients of a certain demographic, etc.; and/or the reverse, only VOAs associated with particular specified patients, only patients of particular specified doctors, and/or only patients of a certain demographic, etc.) or where the user can indicate areas to be removed, e.g., remove from the union or intersection those areas that are also found in VOAs associated with certain specified adverse side effects. The user can similarly select to have a side effect volume returned, formed by the combination (e.g., union or intersection, as specified) of VOAs associated with certain specified adverse side effects.

According to an example embodiment, the system further allows input of multiple logical operators such as a combination of both union and intersection. For example, the user can input an instruction to return a volume formed of the intersection of (a) a first group of one or more VOAs with (b) the union of VOAs of a second group, in response to which the system would find the area formed by the union of the second group, and find the area formed by the intersection of that union area with the area within the VOAs of the first group.

Thus, the system provides a user interface which includes a toolset by which a user can define how the system generates new volumes which the user can then use, e.g., for a current patient, for example, to set stimulation parameters of a current patient.

## 13. Other Analyses:

SFM analyses can include analysis of variance (ANOVA), generalized linear models, parametric or non-parametric techniques, Bayesian analysis, etc.

## Obtaining Analysis Paradigms and/or Data for Analysis

According to an example embodiment of the present invention, the system includes features by which to collect VOA related data over time to then be subject to an analysis, for example, one or more of the analyses described above. According to an example embodiment, the system is configured such that, where a user begins compilation of a parameter set of an analysis to be conducted on an input set of data, e.g., an input set of VOAs, as described above, the user is able to save the constructed analysis paradigm and retrieve it a later time, e.g., for modification and/or application to a set of data input. In an example embodiment, a saved analysis paradigm may function as a template, e.g., which can be copied as a new analysis paradigm, which copy can be further modified. Moreover, the data collection to which an analysis is applied can be a parameter of a saved analysis paradigm. A template can be copied multiple times, and modified to be applied to different sets of data.

In an example embodiment, an analysis template can be saved without specification of a data collection to which the analysis is to be applied. Such a template can be copied as a new analysis record and modified to specify the data collection on which the analysis is performed and to include results of such an analysis. Multiple copies can be saved, each specifying, for example, a different data collection.

For example, an analysis paradigm can be set up by which to find spatial differences between two different types of groups, Group A data set and Group B data set. Further, the analysis can be set with, for example, certain thresholds to rate the data (e.g., threshold overlap or threshold difference), certain statistical tests to be applied, etc. Thus, there can be a number of parameters to use for an analysis. Such a paradigm specifying one or more of such variable parameters can be saved as a template, and copied as new analysis paradigms to which to apply different data sets or modified analysis parameters, for which a user can select an activation instruction, in response to which the system runs the modified analysis on the respectively specified data. For example, the system may display a "run" button in a GUI, in response to selection of which, the system runs the specified analysis.

According to an example embodiment, the templates can be stored as a data structure that can be shared by users. For example, in an example embodiment, a template can be attached to an e-mail which one user can send to another user, which other user can open and modify or otherwise use the attached template, e.g.,

where the other user also includes the software adapted to interpret the data structure. Alternatively or additionally, the template can be stored in a central location accessible by a plurality of terminals on which the software is run. In an example embodiment, the data collection, e.g. VOAs and/or associated stimulation parameters, to which the analysis is applied can also be shared e.g., separate from the template and/or as part of the template.

According to an example embodiment of the present invention, the system is configured for storing associated groups of data, e.g., groups of VOAs and/or associated stimulation parameter sets, which groups can be opened by a user to be subjected to various analyses. The groups can further be modified over time. For example, in an example embodiment, the system includes an interface, e.g., a graphical user interface and/or other interface, via which to receive user input for specifying a set of stimulation parameters and/or associated VOAs, and/or via which to output the set of stimulation parameters and/or graphical representations of such VOAs. The system further includes, according to an example embodiment, a selectable menu item, such as an option of a "File" menu selectable from a toolbar, which, when selected allows the user to save the presently open stimulation settings and/or VOA to a database folder representing the group. If no folder representing the group has been previously set up, or if the user otherwise wants a new group, the system allows the user to select "New Folder" or "New Group" or the like to create the folder/group with which to associate the open settings and/or VOA.

In an example embodiment of the present invention, the system displays in a GUI selectable graphical representations of groups that have been previously created, provided for user selection to associate a set of stimulation parameters and/or a VOA that is in focus with the selected group representation. In an example embodiment, the set of settings and/or VOA can be added by drag-and-drop. For example, an icon representing the set of settings and/or VOA that is in focus, e.g., that is displayed, is selectable and can be dropped onto one of the group representations to be included as part of the group. Each stored group can be separately subjected to one or more analyses. In an example embodiment, the system also includes an icon for creating a new group, which is selectable and/or to which a set of stimulation settings and/or a VOA can be dropped, in response to which selection or drop, the system provides for a user interaction by which to name a new group to which the set of settings and/or VOA that is in focus can be added.

For example, as a clinician notices various symptoms associated with a particular VOA, the clinician can use the GUI features to drop the VOA into various "buckets" that can later be used for analysis. For example, the clinician notices eye movement, and therefore associates the VOA in focus with a bucket of VOAs that resulted in eye movement. In this regard, a VOA and/or a set of stimulation settings can be associated with more than one data group, and can be subjected to analyses of such different groups and/or subject to different types of analyses. At any point in time, a user can use the system to subject such a bucket to an analysis, during which the system performs an analysis, e.g., as described herein, on those volumes that are included in the bucket. It is noted that the system can similarly maintain buckets of side effect volumes and target volumes on which analyses can be run.

Various kinds of data can be associated with VOAs (or other volumes), by which the VOAs (or other volumes) can be filtered. Such data can include, for example, diagnosis, age, gender, medication used, clinical test scores, patient assessment of well being, target volume with which the VOA and/or stimulation settings are associated, variance of a medical image of a patient with which the VOA and/or stimulation settings are associated from a standard atlas, quantitative data from a measurement device such as an accelerometer, of a sensor whose signals can have a certain significance, e.g., indicating tremor, straightness of lines, dwell time, etc. According to an example embodiment, a user can filter stored VOAs (or other volume types) and/or sets of stimulation settings by such data, and apply the filtered group to an analysis. For example, with respect to variance between medical image and standard atlas, a user might want to filter out those VOAs and/or settings that are associated with a patient whose medical image(s) varies from a standard atlas by a threshold amount, since results of a stimulation applied to such a patient may be expected to be different than those normally expected from a patient whose anatomy more closely corresponds to the typical anatomical arrangement. Aside from entering filter parameters for obtaining a matching set of volumes to be immediately subjected to an analysis, the system is also configured to provide for filtering of the volumes to obtain a filtered set that can be stored as a new bucket, which can later be retrieved, e.g., for running one or more analyses.

**Creation and Sharing of Target Volumes**

According to an example embodiment, a system is provided that provides for a cycle of testing stimulation settings that produce corresponding VOAs, obtaining results of such tested settings, analyzing such results, selecting a refined target volume based on such analyses, and selecting new stimulation settings to be tested. A refined volume can be selected, e.g., by changing the volume's position, orientation, size, shape, etc. Moreover, in an example embodiment, such modifications can be performed graphically, e.g., by manipulation of graphically displayed nodes.

This cycle can be repeated, e.g., continuously, to refine the stimulation settings. Moreover, the testing can be of stimulation settings of a plurality of patients and the analyses can be of results of such tested settings and/or their corresponding VOAs of a plurality of patients.

For example, information concerning such settings, their corresponding VOAs, and respective results of stimulations using such settings can be stored at a single location for access by one or more clinicians who can set new target volumes and/or choose modified target stimulation regions based on results of the analyses. Figure 6 illustrates the stimulation and analysis cycle by which target volumes and/or stimulation settings can be refined.

Figure 7 shows a modified cycle according to an example embodiment of the present invention, according to which users are able to share and/or publish their discovered and/or input target volumes for implementation by other users. For example, at a step 700, a guide module can transmit stimulation settings to an IPG for application of those settings to electrodes of an implanted leadwire to stimulate an anatomical region of a patient. At step 702 an analysis can be performed on the tested settings, corresponding VOAs, and/or results of such stimulations, tested by one or more clinicians on one or more patients. At step 704, a user can select a new target region based on the analysis, and share it with a community 706 of users, e.g., clinicians, researchers, and/or other users, who can use such a shared target region to select new stimulation settings to test at 700.

Referring to figure 8, according to an example embodiment, at step 800, a first doctor, "Dr. A," uses a Guide module on a workstation, to set stimulation parameters, view a corresponding VOA, apply the settings to an IPG and an implanted electrode leadwire, and/or record results of such stimulation settings.

At step 802, Dr. A selects a target volume based on the results of the applied stimulation settings. (It is noted that a different doctor may instead select the volume. It is also noted that the newly selected volume can be based on analysis of results of settings applied to more than one patient by more than one doctor. It is also noted that the selection of the new target volume can be further based on results of a plurality of different applied sets of stimulation settings. It is also noted that the analysis can be manual or can be automatic, e.g., using one or more of the analyses described above.)

At step 804, the Guide module generates a code for the target volume set by Dr. A. For example, Dr. A selects an option to save the input target volume and the system responsively generates and outputs a code associated with the saved target volume. For example, Dr. A can save the target volume under any descriptive name by which Dr. A can later identify the target volume in a useful manner, but the system can store a field, that includes the generated code, in association with the saved target volume. Further, in an example embodiment, the field can be opened for view by Dr. A so that Dr. A can later identify the code if otherwise forgotten. For example, in an example embodiment, responsive to right-clicking a representation of a file corresponding to the target volume, the user is able to view properties of the file, including the generated code. Alternatively, when the target volume is opened by Dr. A, the system also displays the code. (In an alternative example embodiment, the doctor manually enters a code, and the system is configured to inform the doctor whether the entered code is available.)

At step 806, Dr. A shares the code with one or more other doctors. For example, Dr. A e-mails the code or otherwise publishes the code. At step 808, Dr. B inputs the code into an instantiated Guide module running on Dr. B's terminal, in response to which, at step 810, the Guide module into which Dr. B input the code displays or otherwise uses the target volume selected by Dr. A and for which the code was previously generated. For example, in an example embodiment target volumes are accessible via an alphanumeric code that is published so that other users can then use the code to access a central server that provides them with the target volume, e.g., they can be downloaded via a webpage of the cloud.

In an example embodiment, when other users import such published volumes, the system provides for the importing user to tag the imported volume, for example, with data identifying who generated the volume, in which facility the data was generated, etc., and to store the tagged volume in a folder owned by the importing user. In an alternative embodiment, the system is configured to automatically append such metadata, e.g., which can be accessed by the importing user.

The target volume selected at step 802 can be generated manually by Dr. A, e.g., by manipulation of graphical nodes in a user interface, or can be generated automatically by the system based on input, e.g., selected by Dr. A. For example, Dr. A can input a group including a plurality of sets of stimulations settings, corresponding VOAs, and results into a system-run analysis, e.g., one of the analyses described above, based on target generation parameters (pre-programmed and/or user input) of which the system outputs the target volume, which Dr. A can select for saving.

Dr. A can store a plurality of target volumes. For example, different ones of the stored target volumes can be associated with different groups of patients. For example, different target volumes can be associated with different desired therapeutic effects, different diseases, different indications, etc. In an example embodiment, the system enables the user to identify the characteristic with which the target volume is to be associated. For example, a file name or folder name can be used to identify the characteristic.

While the above discussion concerning sharing of volumes, e.g., in connection with figure 8, has been described with respect to target volumes, in an example embodiment, the system also provides for a user to likewise share side effect volumes. For example, Dr. A can manually enter a side effect region where stimulation is to be avoided or the system can automatically generate a side effect region, e.g., as described above in the "Group Comparisons" section. The system can assign a code to the side effect region, which code can be shared as described above with respect to the target volumes.

In an example embodiment of the present invention, the system stores the user-defined/selected target and/or side effect regions at a central location accessible by a plurality of terminals running a Guide module. It is noted that a number of users can also use a single terminal using different log-in information. The different users of the same or different terminals can thereby obtain, from the central location and via a network, e.g., the Internet, the stored target and/or side effect region previously selected by a different user. The user can identify which volume to obtain by entering the corresponding code.

In an alternative example embodiment, the system generates a code for the selected target or side effect volume based on characteristics of the volume. In an example embodiment, the generation of the code based on the characteristics of the volume is such that the system is able to reconstruct the volume based on the code. For example, the code may be based on one or more of a center of mass of the volume and spatial coordinates of a perimeter of the volume. Other characteristics of the volume as described above with respect to data stored to represent a volume can additionally or alternatively be used. Accordingly, the volume need not be stored. Instead, a user can share a selected volume by sharing the code generated by the system, and another user can enter the shared code, in response to which the system outputs the volume reconstructed based on the code.

In an example embodiment, when a first user, using the Guide software to program a system, enters a code to open a volume shared by a second user, the system is configured to modify the shared volume to reflect an anatomy of the patient. For example, the shared volume might have been generated in a space corresponding to the brain of a different patient or in a generic atlas space, which varies from the anatomical space corresponding to the brain of the currently active patient information.

In an alternative example embodiment the system initially opens the volume according to the spatial environment in which it is saved or according to a generic atlas space (even if the volume was generated in relation to an anatomical space of another patient), and subsequently, in response to a user conversion instruction, transforms the volume to reflect the anatomy of a currently active patient. According to the embodiment in which the code is automatically generated based on characteristics of the volume, in an example embodiment the system is configured to, when a user selects to open the shared volume, open the volume in a generic atlas space, and the user can instruct the system to convert the volume to the patient anatomical space.

The volume being shared can be stored by the system in a manner by which it is not associated with any patient for whom the shared volume was created, in order to preserve the patient's privacy. For example, as noted above, even if the volume is generated in relation to an anatomical space of a patient, the system can be configured to output the shared volume transformed to a generic atlas space. Alternatively, the patient-specific anatomical space can be output since it cannot be used to easily identify the patient.

The system thus facilitates a continuous cycle of refinement of volumes. For example, a first clinician can open a number of target volumes selected by one or more other clinicians based, for example, on similar findings reported by the one or more other clinicians. The user can then have the system run an analysis to find overlapping regions of the multiple target volumes, as discussed above, to thereby form a further refined target volume.

In an example embodiment, the system further includes an option to automatically generate a target volume based on a combination of VOAs that correspond to the multiple selected target volumes. For example, in response to receipt of user input selecting the option to generate the target volume based on the underlying VOAs, the system is configured to find for each of the selected target volumes a best fit set of stimulation variables to provide a respective best fit VOA. The best fit parameter settings and VOA can be patient-specific to a currently active or selected patient. The system then performs the analysis upon the plurality of VOAs to find a new target volume (for which the system is configured to also find a further set of best fit parameter settings and corresponding VOA).

Alternatively, the user can have the system graphically overlap the multiple selected target volumes of the one or more other clinicians or the corresponding best VOAs, and manually outline a new target volume based on the displayed overlap.

## Web Forum

According to an example embodiment, the system includes a server to which system users' volumes are uploaded. A user can create a new web group maintained by the server and invite/add other users to the user-hosted group. Those other users who accept/join the group are then able to view and use volumes shared with the group by other members of the group.

## Target Volume Creation (Moving results to clinic)

Analysis results can be used to generate target (visualization) volumes for both benefits and side-effects. Target volumes can be saved as a mesh or a point (e.g., a centroid with additional information as described above).

In an example embodiment, a target volume is definable by a selected point about which the volume is to be drawn and a volume size. (Angles can further be used to define an orientation of the target volume relative to axes of an anatomical space.) For example, in an example embodiment, the system is configured to identify an average center of mass of a selected plurality of VOAs.

For example, the system is configured to provide a user-selectable option, in response to selection of which the system is configured to calculate the average center of mass of a set of VOAs selected by the user. The system is further configured to receive a size information and draw a target volume centered on the calculated average center of mass and that is of the size specified by the user.

In an alternative example embodiment, or as an additional alternative option, instead of an average center of mass of the VOAs, the system finds a respective average score for each of a plurality of voxels, where the average score for a voxel is an average of the scores of the VOAs in which the respective voxel is included. The score of a VOA can be based on, for example, results of a stimulation to which the VOA corresponds, as described above. In an example embodiment, the system selects the voxel having the highest average score as the point about which to draw the target volume. Alternatively, the system finds a cluster of highest average scoring voxels, and selects the center of such a cluster as the point about which to draw the target volume. The user can manually enter a size, which the user might determine based on a general intuitive feel.

In an alternative example embodiment, or as an additional alternative option, the system first removes from consideration those VOAs having a score below a predetermined programmed threshold, or a threshold specified by the user, and then finds the voxel having the highest average score (or center of a cluster of highest scoring voxels) of the remaining VOAs to set as the point about which to draw the target volume.

In an alternative example embodiment, or as an additional alternative option, the system first removes from consideration those VOAs having a score below a predetermined programmed threshold, or a threshold specified by the user, and then finds the average center of mass of the remaining VOAs to set as the point about which to draw the target volume.

In an alternative example embodiment of the present invention, the system is configured to receive user input of a target volume size (e.g., as number of voxels, a radius, or any other suitable specification of size), in accordance with which size specification the system is configured to adjust a score threshold to one that results in removing just enough voxels to provide a volume approximately equal to the specified size. Alternatively, the system is configured to receive user input of a target volume size (e.g., as number of voxels, a radius, or any other suitable specification of size), in accordance with which size specification the system is configured to adjust a score threshold to one that results in removing just enough voxels to ensure that the specified size is not exceeded. Alternatively, the system is configured to receive user input of a target volume size (e.g., as number of voxels, a radius, or any other suitable specification of size), in accordance with which size specification the system is configured to adjust a score threshold to one that results in removing just enough voxels to ensure that the specified size is not undershot. In an example embodiment, these described methods of adjusting a threshold in accordance with a user-specified volume size are provided as user-selectable options.

The threshold value can be a percentage, e.g., a user may require the target volume to encompass voxels that make up 80% of all the scores of the considered voxels. For example, the system is configured to receive user-input specifying a percentage, and to set the threshold such that the combined average scores of the remaining voxels (whose individual average scores meet the threshold) is equal to the specified percentage of the sum of the average scores of all considered voxels. That is, the voxels of the output volume are such that

$$\frac{\sum_{i=1}^{n\_output} avg\_score\_of\_voxel_i}{\sum_{j=1}^{n\_input} avg\_score\_of\_voxel_j} = x\%$$

, where $i$ is a voxel of the output volume, $n\_output$ is the number voxels in the output volume, $j$ is a voxel of one or more of the input volumes, $n\_input$ is the number of voxels that are included in at least one of the input volumes, and $x$ is the percentage specified by the user.

## Compatibility

In an example embodiment, the system is configured to provide compatibility modes in which to generate and/or analyze VOAs. The system is configured to provide de-featuring in the compatibility modes. For example, data can be scaled down to render the data compatible with third party analysis tools, to allow users to perform analysis from the perspective of the other systems.

In an example compatibility mode, the system turns off the ability to simulate VOAs using multiple independent current or voltage sources, so that only a single source is used for all contacts.

After turning off the relevant features, VOAs can then be generated as if they were done using the hardware and parameters supported by the other systems. Such VOAs can then be applied to a visualization, programming, or analysis tool.

As another example, certain systems allows for leadwire contacts to each be set to either on or off, while other systems allow for leadwire contacts to each be set to a plurality of levels besides for on and off, e.g., 20% power, 30% power, etc. If a user is using a system of the former type, the user can set the Guide and/or analysis modules to a compatibility mode in which contact settings can be set to only on and off, and to lockout features not supported by the used hardware. In an example embodiment, the user is presented with a checklist of features for each of which the user can input whether the feature is supported by the hardware being used.

**Additional Feature:**

**Preop:**

Using the Target and Side Effect regions, and additional constraints, automatically compute an optimal trajectory for implant.

Take the MRI of a plurality of patients and either using the whole MRI or regions of interest within it (e.g., Basal Ganglia), cluster the images using a metric of image similarity. For generating a customized atlas for a new patient, examine to which patients in the database the patient is most similar. The system creates an atlas template for use in the new patient's surgery based in the 'n' closest database MRIs. The atlas can be created by transforming the 'n' closest MRIs to a standard space, e.g., MNI space or it may be created by finding an "average MRI" that best represents the 'n' database MRIs. This may be accomplished using a variety of non-linear registration algorithms. The clustering of the database images can be done hierarchically so as to obtain a cluster tree. At the bottom of the tree are the individual images, and at each branch the system can construct a template utilizing the images below it. This enables an efficient search for similarity between the patient and the candidates.

**Postop:**

Aggregate VOAs across subjects using a standard space (e.g., MNI space) or within a new space which is the space of the average MRI that best represents this subjects MRI.

Enable analytics by allowing clinicians to do "set theory" on the VOAs. For example, clinicians can compute voxels that are common to 1, 2, 3,... VOAs in the database. Each voxel is tagged by all the behavioral scores that its stimulation led to. For example, stimulation of a voxel in one patient may lead to low tremor, in another patient stimulation of the same voxel may lead to high tremor. Physicians can export this data, associating voxels with scores offline for their own analysis, but they can also use the system to do analysis on the information.

For example, users can identify voxels whose activation significantly affects the stimulation using the following method. The system obtains for each voxel a series of scores, in the off (not activated) and on (activated) states. The system determines the difference between average scores during the on and off states. The system permutes the on and off scores randomly and recomputes the difference. This is done 10,000 or so times, and the system plots a histogram of the scores to see where the original difference sits in this histogram. The histogram is the null distribution. If the voxel influences the score significantly, the real difference should be in the tail of the histogram. Definition of the tail requires a threshold, also known as a 'p' value. In an example embodiment, the system provides a user interface by which to specify this threshold (e.g., 0.05, 0.1, etc.) which means that 5% of the values in the null distribution exceed this threshold or 10% exceed this threshold, respectively. The system can display either the voxels that are in the tail according to a user specified threshold or the system can output to the user the 'p' value map itself as a false color 3D map. For example, in an example embodiment, the latter is displayed as false color slices through a 3D space, for example overlayed on the MRI. Other statistical methods can be alternatively used.

According to an example embodiment, the system obtains the activating function in the direction in which it is maximum at each voxel as an independent variable and the behavioral score as an independent variable and determines which voxels show the most robust correlation between the behavioral scores and the activating function magnitude. There are some voxels for which high values of the activating function lead to negative values of the scores relative to baseline (symptoms improve), some voxels for which high values of the activating function lead to positive values relative to baseline (symptoms worsen), and finally some voxels for which the activating function and the symptom improvement are independent of each other. Thus, the system, according to this example embodiment, uses the correlation of the activating function and the scores to determine the effect of voxels on the considered clinical effect.

According to an example embodiment, for each stimulus setting, the electric field distribution and the corresponding activation can be computed using simplified models. If activation of a given region is desired while avoiding affecting another region, an objective function is used that maximizes the overlap with the region of desired activation and minimizes the overlap with the other region. Many objective functions can be used. A difference of the overlaps may be the most efficient. A search in parameter space can be performed to minimize the objective function. Multiple search strategies are possible.

According to an example embodiment of the present invention, a large database of lead contact positions, stimulus parameters, and outcomes are obtained and the system performs a machine learning exercise to model the dependence of stimulus parameters on the outcomes and the contact positions.

The methods and systems described herein may be embodied in many different forms and should not be construed as limited to the embodiments set forth herein. Accordingly, the methods and systems described herein may take the form of an entirely hardware embodiment, an entirely software embodiment or an embodiment combining software and hardware aspects. The methods described herein can be performed using any type of processor or any combination of processors where each processor performs at least part of the process. Systems referenced herein typically include memory and typically include methods for communication with other devices including mobile devices. Methods of communication can include both wired and wireless (e.g., RF, optical, or infrared) communications methods and such methods provide another type of computer readable media; namely communication media. Wired communication can include communication over a twisted pair, coaxial cable, fiber optics, wave guides, or the like, or any combination thereof. Wireless communication can include RF, infrared, acoustic, near field communication, Bluetooth™, or the like, or any combination thereof.

The methods disclosed herein can be implemented by computer program instructions. These program instructions may be provided to a processor to produce a machine, such that the instructions, which execute on the processor, create means for implementing the actions. The computer program instructions may be executed by a processor to cause a series of operational steps to be performed by the processor to produce a computer implemented process. The computer program instructions may also cause at least some of the operational steps to be performed in parallel. Moreover, some of the steps may also be performed across more than one processor, such as might arise in a multiprocessor computer system. In addition, one or more processes may also be performed concurrently with other processes, or even in a different sequence than illustrated.

The above description is intended to be illustrative, and not restrictive. Those skilled in the art can appreciate from the foregoing description that the present invention may be implemented in a variety of forms, and that the various embodiments may be implemented alone or in combination, as defined by the appended claims.

## Claims

1. A computer-implemented method, comprising:

   obtaining user input of a clinical effect, wherein the clinical effect is either a therapeutic effect or a side effect;
   obtaining a plurality of estimated volumes of activation (VOA) associated with stimulations of a patient population, wherein a first group of the VOAs is associated with the clinical effect and a second group of the VOAs is not associated with the clinical effect;
   based on the VOAs, determining a target stimulation region, when the clinical effect is the therapeutic effect, or a side effect region, when the clinical effect is the side effect, by determining, on a voxel-by-voxel basis, that a voxel is in the target stimulation region when the voxel is included in statistically significantly more of the VOAs of the first group of VOAs than in the VOAs of the second group of VOAs; and
   outputting the determined target stimulation or side effect region.

2. The computer-implemented method of claim 1, further comprising transforming all of the VOAs into a common spatial reference space.

3. The computer-implemented method of claim 1, further comprising filtering the VOAs by a time of day when electrical stimulation of each VOA was performed.

4. The computer-implemented method of claim 1, further comprising filtering the VOAs by a medication used by a patient associated with each VOA.

5. The computer-implemented method of claim 1, further comprising generating a probability voxel map that maps a probability that stimulation of each respective voxel of the probability voxel map will contribute to the clinical effect.

6. The computer-implemented method of claim 1, further comprising, for the first group of the VOAs, determining an overlap of a significant number of the VOAs with a shared component of a biological circuit element.

7. The computer-implemented method of claim 6, further comprising, based on a degree of the overlap, outputting an indication of the biological circuit element as associate with the clinical effect.

8. The computer-implemented method of claim 6, further comprising generating a new target stimulation region, when the clinical effect is therapeutic effect, or new side effect region, when the clinical effect is a side effect, based on the VOAs which overlap the biological circuit component.

9. The computer-implemented method of claim 6, further comprising displaying a biological circuit that includes the biological circuit element.

10. The computer-implemented method of claim 1, further comprising filtering the VOAs to include only VOAs in the first and second groups that correspond to patients having a same medication.

11. The computer-implemented method of claim 1, further comprising filtering the VOAs to include only VOAs in the first and second groups that correspond to patients having a same medication and dosage.

12. The computer-implemented method of claim 1, further comprising filtering the VOAs to include only VOAs in the first and second groups that correspond to stimulations performed at a same period in the day.

13. The computer implemented method of claim 12, further comprising analyzing the VOAs of the first group based on a time of day that stimulation occurred to determine a target stimulation time.

14. The computer implemented method of claim 13, further comprising creating a histogram of stimulations associated with the VOAs of the first group by time of day.

15. The computer implemented method of claim 1, wherein the method is performed separately for both a therapeutic effect and a side effect to determine a target stimulation region and a side effect region, respectively.

**Patentansprüche**

1. Computerimplementiertes Verfahren, das aufweist:

Erhalten einer Benutzereingabe einer klinischen Wirkung, wobei die klinische Wirkung entweder eine therapeutische Wirkung oder eine Nebenwirkung ist;
Erhalten mehrerer geschätzter Aktivierungsvolumina (VOA), die Stimulationen einer Patientenpopulation zugeordnet sind, wobei eine erste Gruppe der VOAs der klinischen Wirkung zugeordnet ist und eine zweite Gruppe der VOAs nicht der klinischen Wirkung zugeordnet ist;
Bestimmen eines Ziel-Stimulationsbereichs, wenn die klinische Wirkung die therapeutische Wirkung ist, oder eines Nebenwirkungsbereichs, wenn die klinische Wirkung die Nebenwirkung ist, basierend auf den VOAs durch Bestimmen, auf einer Voxel-für-Voxel-Basis, dass ein Voxel innerhalb des Ziel-Stimulationsbereichs liegt, wenn das Voxel in statistisch signifikant mehr VOAs der ersten Gruppe von VOAs als in den VOAs der zweiten Gruppe von VOAs enthalten ist; und
Ausgeben des bestimmten Ziel-Stimulationsbereichs oder Nebenwirkungsbereichs.

2. Computerimplementiertes Verfahren nach Anspruch 1, ferner mit Transformieren aller VOAs in einen gemeinsamen räumlichen Bezugsraum.

3. Computerimplementiertes Verfahren nach Anspruch 1, ferner mit Filtern der VOAs nach einer Tageszeit, zu der eine elektrische Stimulation jedes VOA ausgeführt wurde.

4. Computerimplementiertes Verfahren nach Anspruch 1, ferner mit Filtern der VOAs nach einer Medikation, die durch einen Patienten angewendet wird, der jedem VOA zugeordnet ist.

5. Computerimplementiertes Verfahren nach Anspruch 1, ferner mit Erzeugen einer Wahrscheinlichkeits-Voxelkarte, die eine Wahrscheinlichkeit abbildet, dass die Stimulation jedes jeweiligen Voxels der Wahrscheinlichkeits-Voxelkarte zu der klinischen Wirkung beiträgt.

6. Computerimplementiertes Verfahren nach Anspruch 1, ferner mit Bestimmen einer Überlappung einer signifikanten Anzahl der VOAs mit einer gemeinsamen Komponente eines biologischen Schaltkreiselements für die erste Gruppe der VOAs.

7. Computerimplementiertes Verfahren nach Anspruch 6, ferner mit dem Ausgeben einer Anzeige des biologischen Schaltkreiselements als der klinischen Wirkung zugeordnet basierend auf einem Grad der Überlappung.

8. Computerimplementiertes Verfahren nach Anspruch 6, ferner mit Erzeugen eines neuen Ziel-Stimulationsbereichs, wenn die klinische Wirkung eine therapeutische Wirkung ist, oder eines neuen Nebenwirkungsbereichs, wenn die klinische Wirkung eine Nebenwirkung ist, basierend auf den VOAs, die das biologische Schaltkreiselement überlappen.

9. Computerimplementiertes Verfahren nach Anspruch 6, ferner mit Darstellen eines biologischen Schaltkreises, der das biologische Schaltkreiselement enthält.

10. Computerimplementiertes Verfahren nach Anspruch 1, ferner mit Filtern der VOAs zum Erfassen nur derjenigen VOAs in der ersten und in der zweiten Gruppe, die Patienten entsprechen, die eine gleiche Medikation erhalten.

11. Computerimplementiertes Verfahren nach Anspruch 1, ferner mit Filtern der VOAs zum Erfassen nur derjenigen VOAs in der ersten und in der zweiten Gruppe, die Patienten entsprechen, die eine gleiche Medikation und Dosierung erhalten.

12. Computerimplementiertes Verfahren nach Anspruch 1, ferner mit Filtern der VOAs zum Erfassen nur derjenigen VOAs in der ersten und in der zweiten Gruppe, die Stimulationen entsprechen, die zu einer gleichen Tageszeit ausgeführt werden.

13. Computerimplementiertes Verfahren nach Anspruch 12, ferner mit Analysieren der VOAs der ersten Gruppe basierend auf einer Tageszeit, zu der die Stimulation erfolgte, zum Bestimmen einer Soll-Stimulationszeit.

14. Computerimplementiertes Verfahren nach Anspruch 13, ferner mit Erstellen eines Histogramms von Stimulationen, die den VOAs der ersten Gruppe zugeordnet sind, gemäß der Tageszeit.

15. Computerimplementiertes Verfahren nach Anspruch 1, wobei das Verfahren sowohl für eine therapeutische Wirkung als auch für eine Nebenwirkung getrennt ausgeführt wird, zum Bestimmen eines Ziel-Stimulationsbereichs bzw. eines Nebenwirkungsbereichs.

**Revendications**

1. Procédé mis en oeuvre par ordinateur, comprenant les étapes ci-dessous consistant à :

obtenir une entrée utilisateur d'un effet clinique, dans lequel l'effet clinique est soit un effet thérapeutique, soit un effet secondaire ;
obtenir une pluralité de volumes d'activation (VOA) estimés associés à des stimulations d'une population de patients, dans lequel un premier groupe des volumes VOA est associé à l'effet clinique et un second groupe des volumes VOA n'est pas associé à l'effet clinique ;
sur la base des volumes VOA, déterminer une région de stimulation cible, lorsque l'effet clinique est l'effet thérapeutique, ou une région d'effet secondaire, lorsque l'effet clinique est l'effet secondaire, en déterminant, sur une base « voxel par voxel », qu'un voxel se situe dans la région de stimulation cible lorsque le voxel est inclus dans un nombre statistiquement significativement plus élevé des volumes VOA du premier groupe des volumes VOA que des volumes VOA du second groupe des volumes VOA ; et
fournir en sortie la région de stimulation cible déterminée ou la région d'effet secondaire déterminée.

2. Procédé mis en oeuvre par ordinateur selon la revendication 1, comprenant en outre l'étape consistant à transformer la totalité des volumes VOA en un espace de référence spatial commun.

3. Procédé mis en oeuvre par ordinateur selon la revendication 1, comprenant en outre l'étape consistant à filtrer les volumes VOA en fonction d'une heure de la journée où la stimulation électrique de chaque volume VOA a été mise en oeuvre.

4. Procédé mis en oeuvre par ordinateur selon la revendication 1, comprenant en outre l'étape consistant à filtrer les volumes VOA en fonction d'un médicament utilisé par un patient associé à chaque volume VOA.

5. Procédé mis en oeuvre par ordinateur selon la revendication 1, comprenant en outre l'étape consistant à générer une carte de voxels de probabilité qui cartographie une probabilité que la stimulation de chaque voxel respectif de la carte de voxels de probabilité contribuera à l'effet clinique.

6. Procédé mis en oeuvre par ordinateur selon la revendication 1, comprenant en outre, pour le premier groupe des volumes VOA, l'étape consistant à déterminer un chevauchement d'un nombre significatif des volumes VOA avec un composant partagé d'un élément de circuit biologique.

7. Procédé mis en oeuvre par ordinateur selon la revendication 6, comprenant en outre, sur la base d'un degré du chevauchement, l'étape consistant à fournir en sortie une indication selon laquelle l'élément de circuit biologique est associé à l'effet clinique.

8. Procédé mis en oeuvre par ordinateur selon la revendication 6, comprenant en outre l'étape consistant à générer une nouvelle région de stimulation cible, lorsque l'effet clinique est un effet thérapeutique, ou une nouvelle région d'effet secondaire, lorsque l'effet clinique est un effet secondaire, sur la base des volumes VOA qui chevauchent l'élément de circuit biologique.

9. Procédé mis en oeuvre par ordinateur selon la revendication 6, comprenant en outre l'étape consistant à afficher un circuit biologique qui inclut l'élément de circuit biologique.

10. Procédé mis en oeuvre par ordinateur selon la revendication 1, comprenant en outre l'étape consistant à filtrer les volumes VOA pour n'inclure que des volumes VOA, dans le premier groupe et le second groupe, qui correspondent à des patients ayant un même médicament.

11. Procédé mis en oeuvre par ordinateur selon la revendication 1, comprenant en outre l'étape consistant à filtrer les volumes VOA pour n'inclure que des volumes VOA, dans le premier groupe et le second groupe, qui correspondent à des patients ayant un même médicament et un même dosage.

12. Procédé mis en oeuvre par ordinateur selon la revendication 1, comprenant en outre l'étape consistant à filtrer les volumes VOA pour n'inclure que des volumes VOA, dans le premier groupe et le second groupe, qui correspondent à des stimulations mises en oeuvre au cours d'une même période de la journée.

13. Procédé mis en oeuvre par ordinateur selon la revendication 12, comprenant en outre l'étape consistant à analyser les volumes VOA du premier groupe sur la base d'une heure de la journée où la stimulation a eu lieu, en vue de déterminer une heure de stimulation cible.

**14.** Procédé mis en oeuvre par ordinateur selon la revendication 13, comprenant en outre l'étape consistant à créer un histogramme de stimulations associées aux volumes VOA du premier groupe en fonction de l'heure de la journée.

**15.** Procédé mis en oeuvre par ordinateur selon la revendication 1, dans lequel le procédé est mis en oeuvre séparément, tant pour un effet thérapeutique que pour un effet secondaire, en vue de déterminer une région de stimulation cible et une région d'effet secondaire, respectivement.

Figure 1

Figure 9

Clinician Programmer Terminal

202

IPG

200

100

Figure 2

Clinician Programmer Terminal

202

IPG

200

100

Figure 3

Obtain
target VOA                     400

↓

Determine
sets of
stimulation                    402
settings

↓

| Set IPG(s)        404 | Obtain patient condition information        406 |

↓

Score sets
of
stimulation                    408
settings

↓

Select best
scoring                        410
set(s)

Figure 4

Amplitude

Pulse Width

Figure 5

602

Clinical
(see and place
stimulation)

606

Target
Volumes

604

Analytical
(understand
stimulation)

Figure 6

700

Clinical:
Perform
stimulation

706

Guide Community

702

Analysis

Create and share to
community

704

Figure 7

Apply stimulation
parameters
800

NEJM

Input code
808

Select target
volume
802

E-mail, publish,
etc., target volume
code from Guide
806

Guide
uses code
810

Generate code
804

Figure 8

Tremor
Improvement
Path 1104

Current VOA
1100

Optimal VOA
1102

Figure 11

A

Figure 10

Figure 12

Figure 13

STUDY Collaboration:
Most effective contacts
relative to STN fill

Surgical
Planning

Atlas + Ref Volume to Surgical
Planning

Programming
(GUIDE)

AC/PC/MSP to Program Planning
Module

STN fill, surgeon-recommended
target to Program Planning Module

Atlas Transformation to Program
Planning Module

Clinical
Effects Data

Figure 14

Figure 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 45433009
- US 45431209
- US 45434009
- US 45434309
- US 45431409
- US 61468884
- US 61468887
- US 61468891
- US 61468897
- US 61468901
- US 571078
- US 13570998 B
- US 61521626
- US 61521641 B
- US 61521632 B
- US 61676000 B
- US 61676014 B
- US 43123212
- US 61468884 B
- US 61468887 B
- US 61468891 B
- US 61468897 B
- US 61468901 B
- US 21273014
- US 61793773 B
- US 61830855 B
- US 60085512
- US 01183613
- US 61693866 B
- US 61699135 B
- US 61699115 B
- US 61753232 B
- US 61904248 B
- US 61521572 A
- US 61549053 B
- US 57073612
- US 61651282 B
- US 61521572 B

### Non-patent literature cited in the description

- **SIEGEL et al.** Spatiotemporal Dynamics of the Functional Architecture for Gain Fields in Inferior Parietal Lobule of Behaving Monkey. *Cerebral Cortex,* February 2007, vol. 17 (2), 378-390